# FASCICULE DE BREVET EUROPEEN

(11) **EP 3 759 161 B1**
(45) Date de publication et mention de la délivrance du brevet: **01.11.2023**
(21) Numéro de dépôt: 19706702.8
(22) Date de dépôt: 27.02.2019
(51) Int. Cl.: C08J 9/36, B81C 1/00, B82Y 30/00, C12N 11/08, C08J 7/04, C08J 9/00

(54) **PROCÉDÉ DE DÉPÔT DE NANOOBJETS À LA SURFACE D'UN GEL POLYMÉRIQUE COMPRENANT DES ZONES DE RIGIDITÉS DISTINCTES**
VERFAHREN ZUR ABSCHEIDUNG VON NANOOBJEKTEN AUF DER OBERFLÄCHE EINES POLYMERGELS MIT ZONEN MIT UNTERSCHIEDLICHEN STEIFIGKEITEN
METHOD FOR DEPOSITING NANO-OBJECTS ON THE SURFACE OF A POLYMER GEL COMPRISING ZONES WITH DISTINCT RIGIDITIES

(30) Priorité: 02.03.2018 FR 1851833
(43) Date de publication de la demande: 06.01.2021
(73) Titulaire: Centre National de la Recherche Scientifique, 75016 Paris (FR); Université Grenoble Alpes, 38400 Saint-Martin-d'Hères (FR); Commissariat à l'Énergie Atomique et aux Énergies Alternatives, 75015 Paris (FR)
(72) Inventeur: NICOLAS, Alice, 38054 Grenoble Cedex 9 (FR); MIGDAL, Camille, 38054 Grenoble Cedex 9 (FR); LOPEZ SOLER, Eline, 38054 Grenoble Cedex 9 (FR)
(74) Mandataire: Brevalex
(86) Numéro de dépôt international: PCT/EP2019/054859
(87) Numéro de publication internationale: WO 2019/166487

(56) Documents cités:
- FR-A1- 2 983 201
- TSE J R ET AL: "Preparation of Hydrogel Substrates with Tunable Mechanical Properties", CURRENT PROTOCOLS IN CELL BIOLOGY, JOHN WILEY & SONS, INC, US, vol. 47, no. Suppl. 47, 1 juin 2010 (2010-06-01), pages 10.16.1-10.16.6, XP002679865, ISSN: 1934-2500, DOI: 10.1002/0471143030.CB1016S47 [extrait le 2010-06-01] cité dans la demande
- ZAARI N ET AL: "PHOTOPOLYMERIZATION IN MICROFLUIDIC GRADIENT GENERATORS: MICROSCALE CONTROL OF SUBSTRATE COMPLIANCE TO MANIPULATE CELL RESPONSE", ADVANCED MATERIALS, WILEY-VCH GERMANY, DE, vol. 16, no. 23-24, 1 décembre 2004 (2004-12-01), pages 2133-2137, XP002679866, ISSN: 0935-9648, DOI: 10.1002/ADMA.200400883 [extrait le 2004-12-16] cité dans la demande
- KLOXIN A M ET AL: "In situ elasticity modulation with dynamic substrates to direct cell phenotype", BIOMATERIALS, ELSEVIER SCIENCE PUBLISHERS BV., BARKING, GB, vol. 31, no. 1, 1 janvier 2010 (2010-01-01), pages 1-8, XP026719454, ISSN: 0142-9612, DOI: 10.1016/J.BIOMATERIALS.2009.09.025 [extrait le 2009-09-27] cité dans la demande
- WONG JOYCE Y ET AL: "Directed movement of vascular smooth muscle cells on gradient-compliant hydrogels", LANGMUIR, AMERICAN CHEMICAL SOCIETY, US, vol. 19, no. 5, 1 janvier 2003 (2003-01-01), pages 1908-1913, XP002513302, ISSN: 0743-7463, DOI: 10.1021/LA026403P [extrait le 2003-01-09] cité dans la demande

## Description

La présente invention concerne un procédé de dépôt de nanoobjets à la surface d'un gel polymérique comprenant des zones de rigidités distinctes, ce procédé permettant avantageusement d'obtenir des densités surfaciques en nanoobjets distinctes, où la densité surfacique en nanoobjets des zones les plus rigides est supérieure à celle des zones les moins rigides, le gel susceptible d'être obtenu et ses applications.

La demande WO 2013/079231 et les articles de Tse et al., Current Protocols in Cell Biology, 47, 2010, 10.16-1-10.6-16, de Zaari et al., Adv. mater. 16, 23-24, 2004, de Kloxin et al. Biomaterials, 31(1), 2010, 1-8 et de Wong et al., Langmuir, 19(5), 2003, 1908-1913 décrivent des procédés de préparation d'hydrogels comprenant des zones de rigidités distinctes et dont la surface est greffée avec des protéines puis sur laquelle des cellules sont déposées. Ces procédés sont exempts d'étape d'évaporation de l'eau des hydrogels. Un des objectifs de ces documents est d'étudier la migration, l'étalement et/ou la différentiation des cellules à la surface de l'hydrogel selon la rigidité de ses zones. Les migrations des cellules, qui sont vivantes, sont induites par le gradient mécanique présent à la surface de l'hydrogel. En effet, ces documents enseignent que les protéines sont greffées de façon uniforme sur la surface de l'hydrogel, et qu'il n'y a donc pas de gradient de chimie de surface.

Dans de nombreux domaines, en particulier en biologie, pharmaceutique, diagnostic et dans le domaine des capteurs, des dispositifs comprenant un substrat dont la surface comprend des nanoobjets (protéines, nanoparticules...) disposés de façon localisée et ayant donc une densité surfacique variable sont recherchés. Un gradient de chimie de surface est alors recherché.

Il est relativement aisé de déposer localement des nanoobjets à la surface d'un substrat "dur" comme le verre ou le silicium. Toutefois, afin de renforcer les forces de liaison entre la surface et les nanoobjets et ainsi de prolonger la durée de vie du dispositif, on cherche à remplacer le verre ou le silicium par des substrats plus mous, comme par des gels à base de matrice polymériques, tels que les hydrogels.

La densité d'un gel à base d'une matrice polymérique est directement liée à sa porosité. Plus un gel est poreux, moins il est rigide, et réciproquement. Dans un gel comprenant des zones de rigidités distinctes, les zones les plus molles sont les plus poreuses, et les zones les plus rigides sont les moins poreuses.

La littérature rapporte des méthodes pour organiser des nanoobjets sur des substrats de rigidité/porosité uniforme :
- sur des substrats de type hydrogels: (i) par tamponnage, (ii) par dépôt des molécules à travers un pochoir (iii) à l'aide d'un dispenseur de microgouttes, ou (iv) par activation de la surface de l'hydrogel par une irradiation à travers un photomasque ou par un laser.
- sur des substrats qui ne gonflent pas dans l'eau, type élastomères, verre, silicium, métaux: par auto-assemblage dirigé par les interactions en jeu lors du démouillage d'un film de solution colloïdale, par évaporation d'une émulsion, par évaporation d'un assemblage organisé par une force magnétique ou optique, par auto-assemblage par évaporation dans un substrat texturé ou par tamponnage.

Mais ces méthodes ne se transposent pas nécessairement facilement à des substrats de rigidité variable, ou s'ils sont transposables, il n'y a aucun lien entre les zones sur lesquelles les nanoobjets sont déposés et la rigidité du gel.

La modulation spatiale de l'affinité chimique de la surface d'hydrogels est un enjeu dans le domaine pharmaceutique, où des puces à cellules sont fabriquées afin de tester l'efficacité de drogues sur une population cellulaire placée dans des conditions contrôlées et reproductibles les plus pertinentes possibles au regard des conditions physiologiques. En sus de la chimie de la surface d'adhérence et de la géométrie imposée aux cellules, un paramètre clé est la rigidité du substrat qui doit s'approcher des rigidités physiologiques (0.1 à 100 kPa). Les hydrogels sont les seuls matériaux couvrant cette gamme de rigidité. Dans ce contexte, il est particulièrement recherché de pouvoir organiser des protéines selon des motifs choisis sur un hydrogel de rigidité choisie.

Des techniques de greffage local de protéines par des méthodes de tamponnage ou de pochoir ont été décrites, mais elles sont très limitées en ce qu'elles ne sont pas très adaptées au greffage sur des surfaces molles comme des hydrogels. Le développement de méthodes alternatives de greffage local est donc requis.

Dans le domaine du diagnostic, des puces de capture à biomolécules sont utilisées afin de sonder le contenu de solutions biologiques ou de sérum. Certaines puces utilisent comme substrat des gels à base de matrice polymérique ou des hydrogels (« Arrayit Microarray hydrogel protein substrates », « Reichert Sensor Chips » par exemple) car ces derniers permettent une adsorption non spécifique plus efficace et robuste de la molécule sonde qu'une surface de verre, et ce, d'autant plus que le substrat est poreux. Le dépôt des molécules sondes est fait à l'aide d'un dispenseur de microgouttes. Il est toutefois très délicat que tous les dépôts aient des compositions identiques. Par ailleurs, la taille des dépôts est micrométrique et uniforme sur toute la puce, le profil du dépôt est circulaire. Cette méthode ne peut donc pas être utilisée lorsque des dépôts de formes variables en géométrie ou en taille sont souhaités.

Dans le domaine des capteurs, l'organisation de nanoparticules en motifs est un moyen de faire de la détection ultrasensible d'analytes par détection plasmonique, de faire de la chimie de surface localisée sur des particules pré-assemblées à façon (chimie des thiols en particulier), ou de fabriquer des sondes locales de pH ou de température lorsque les nanoparticules sont déposées sur un gels à base de matrice polymérique tel qu'un hydrogel. L'assemblage en motifs de nanoparticules est aussi un moyen de miniaturiser des composants opto électroniques tels que des générateurs de lumière.

Les méthodes existantes pour déposer des nanoobjets à la surface de gels dont la surface comprend des motifs utilisent des stratégies d'évaporation dirigée d'une solution colloïdale de nanoparticules pour former les organisations souhaitées, telles que Langmuir Blodgett, utilisation d'un pochoir, organisation préliminaire sous champ électrique ou magnétique, organisation préliminaire en 3D par le contrôle des interactions physico-chimiques. L'étape délicate d'évaporation dirigée est toutefois très délicate à mettre en oeuvre.

Des méthodes alternatives pour organiser des nanoobjets sur des substrats de rigidité/porosité variable sont donc requises.

A cet effet, selon un premier objet, l'invention concerne un procédé de dépôt de nanoobjets sur la surface d'un gel comprenant une matrice polymérique comprenant au moins deux zones contiguës de rigidités distinctes, ledit procédé comprenant les étapes de :
a) fournir un gel comprenant une matrice polymérique et un solvant au sein de la matrice polymérique, la matrice polymérique formant un réseau tridimensionnel susceptible de gonfler en présence dudit solvant, où la solubilité de la matrice polymérique à 1 bar et 25°C dans le solvant est inférieure à 1 g/L, la matrice polymérique comprenant au moins deux zones contiguës de rigidités distinctes présentant un gradient de rigidité supérieur ou égal à 0,1 kPa/µm, puis
b) déposer des nanoobjets, tels que définis dans la revendication 1, à la surface du gel, puis
c) évaporer le solvant du gel au moins jusqu'à ce que la variation du taux d'évaporation du solvant de la au moins une zone la moins rigide du gel ne soit pas constante dans le temps, ce par quoi les nanoobjets migrent vers la au moins une zone la plus rigide du gel et un gel est obtenu dont la surface est au moins partiellement revêtue de nanoobjets, et où la densité surfacique en nanoobjets d'au moins une zone la plus rigide parmi les au moins deux zones contiguës est supérieure à celle d'au moins une zone la moins rigide parmi les au moins deux zones contiguës.

Le procédé comprend une étape a) de fourniture d'un gel comprenant une matrice polymérique et un solvant au sein de la matrice polymérique, la matrice polymérique formant un réseau tridimensionnel susceptible de gonfler en présence dudit solvant, où la solubilité de la matrice polymérique à 1 bar et 25°C dans le solvant est inférieure à 1 g/L.

Le gel comprend une matrice polymérique et un solvant au sein de la matrice polymérique, la matrice polymérique formant un réseau tridimensionnel susceptible de gonfler en présence dudit solvant. La matrice polymérique est donc capable de retenir une proportion de solvant au sein de sa structure. Généralement, la teneur maximale en solvant au sein de la matrice polymérique du gel à 25°C (calculée comme le ratio entre le poids de solvant maximal par rapport à la somme du poids de solvant maximal et du poids de la matrice polymérique sèche) varie de 20 à 100%, de préférence de 38 à 100%. Quand on continue à ajouter du solvant au-delà de la teneur maximale, le solvant ajouté n'est plus incorporé au sein de la matrice polymérique.

Le polymère de la matrice polymérique du gel peut être homopolymérique (réseau tridimensionnel formé à partir d'un homopolymère), copolymérique (réseau tridimensionnel formé à partir d'un copolymère) ou multipolymérique (réseau tridimensionnel de polymères interpénétrant (« interpenetrating polymeric gel » (IPN) en anglais).

Généralement, la matrice polymérique comprend (voire est constituée de) un polymère choisi parmi :
- les polyacrylamides ;
- les polyéthylène glycols, polypropylène glycols et copolymères d'éthylène glycol ou de propylène glycol, ceux-ci comprenant éventuellement des motifs issus de la polymérisation de composés (meth)acrylates;
- les polysaccharides, comprenant éventuellement des motifs répétitifs issus de la polymérisation de composés (meth)acrylates) ;
- les (co)polymères issus de la polymérisation de composés diacrylates et/ou (méth)acrylates ;
- les alcools polyvinyliques comprenant des motifs répétitifs issus de la polymérisation de composés (meth)acrylates;
- les dextranes comprenant des motifs répétitifs issus de la polymérisation de composés (méth)acrylates ;
- les polyfumarates de propylène et les poly (fumarate de propylène-co-éthylène glycol);
- les polysiloxanes, comme le poly(dimethylsiloxane) (PDMS) ; et
- les combinaisons de ceux-ci.

Les matrices polymériques à base de polyacrylamides, et en particulier issus de la polymérisation de l'acrylamide et du N,N'-méthylènebisacrylamide, sont particulièrement préférées

Par « composés (meth)acrylates », on entend des composés dérivés d'acrylate ou de méthacrylate, par exemple choisis parmi l'acide acrylique (AA), l'acide méthacrylique (MA), le diméthacrylate d'éthylène glycol (EGDMA), le méthacrylate de 2-hydroxyéthyle (HEMA), l'acrylate de sulfopropyle, où les acides peuvent être sous forme de sel, notamment de sodium ou de potassium.

Le solvant peut être tout solvant dans lequel la solubilité de la matrice polymérique à 1 bar et 25°C est inférieure à 1 g/L et dans lequel elle est susceptible de gonfler.

Par exemple, le solvant peut être une solution aqueuse ou un solvant organique choisi parmi les alcools, les alcanes (pentane, hexane par exemple), les amines (triéthylamine, diisopropylamine par exemple), les cétones (l'acétone par exemple) et les solvants aromatiques (toluène, xylène par exemple).

Dans un mode de réalisation, la matrice polymérique comprend (voire est constituée de) polysiloxanes, comme le poly(dimethylsiloxane) (PDMS), et le solvant est choisi parmi le pentane, la triéthylamine, la diisopropylamine ou le xylène.

Le solvant le plus usuel est une solution aqueuse. Le gel est alors un hydrogel. Des exemples d'hydrogel sont fournis dans la revue de Enas M. Ahmed (Journal of Advanced Research, 2015, 6, 105-121). La matrice polymérique comprend alors généralement (voire est constituée de) un polymère choisi parmi :
- les polyacrylamides, par exemple issues de la polymérisation de l'acrylamide et du N,N'-méthylènebisacrylamide ;
- les polyéthylène glycols, polypropylène glycols et copolymères d'éthylène glycol ou de propylène glycol, ceux-ci comprenant éventuellement des motifs issus de la polymérisation de composés (meth)acrylates;
- les polysaccharides, comprenant éventuellement des motifs répétitifs issus de la polymérisation de composés (meth)acrylates) ;
- les (co)polymères issus de la polymérisation de composés diacrylates et/ou (méth)acrylates ;
- les alcools polyvinyliques comprenant des motifs répétitifs issus de la polymérisation de composés (meth)acrylates;
- les dextranes comprenant des motifs répétitifs issus de la polymérisation de composés (méth)acrylates ;
- les polyfumarates de propylène et les poly (fumarate de propylène-co-éthylène glycol) ; et
- les combinaisons de ceux-ci.

Le solvant peut comprendre un agent viscosifiant, par exemple du glycérol. Cet agent permet d'augmenter la viscosité du solvant et donc de limiter son évaporation.

La matrice polymérique du gel utilisé dans le procédé comprend au moins deux zones contiguës de rigidités distinctes présentant un gradient de rigidité supérieur ou égal à 0,1 kPa/µm, généralement de l'ordre de 1 kPa/µm.

La rigidité de chaque zone est généralement de 100 Pa à 500 kPa, notamment de 0,2 kPa à 100 kPa, de préférence de 0,5 kPa à 50 kPa. Par exemple, le(es) zone(s) la(es) plus rigide(s) a(ont) une rigidité de 5 kPa à 100 kPa, de préférence de 5 kPa à 50 kPa et/ou la(es) zone(s) la(es) moins rigide(s) a(ont) une rigidité de 0,2 kPa à 10 kPa, de préférence de 0,5 kPa à 5 kPa.

La rigidité locale de chaque zone de la matrice polymérique et le gradient de rigidité entre deux zones contiguës peuvent être déterminés par microscopie à force atomique (AFM), par exemple en suivant le protocole décrit pages 29 et 30 de la demande WO 2013/079231. Le gradient de rigidité est mesuré sur la surface de la zone sur laquelle seront déposés les nanoobjets lors de l'étape b).

Généralement, la plus rigide des au moins deux zones contiguës se présente sous la forme d'un motif. Entre un motif moins rigide à une zone plus rigide, il peut y avoir des paliers de rigidité croissante, comme des « marches d'escalier ». De même, entre un motif plus rigide et une zone moins rigide, il peut y avoir des paliers de rigidité décroissante. La frontière entre les régions moins rigide et plus rigide est généralement une droite ou un cercle. Avantageusement, la géométrie du(es) motif(s) est à façon (carrée, circulaire, triangulaire, hexagonale...). Sa taille peut aller de quelques centaines de nanomètres à plusieurs centimètres, typiquement de 10 µm à 10 mm.

La surface du gel peut comprendre une alternance de motifs rigides et de motifs mous. En alternative, la surface du gel peut comprendre plusieurs motifs rigides localisés dans une matrice continue molle.

Des hydrogels dont la matrice polymérique comprenant au moins deux zones contiguës de rigidités distinctes présentant un gradient de rigidité supérieur ou égal à 0,1 kPa/µm peuvent par exemple être préparés par photopolymérisation en suivant le procédé décrit dans la demande WO 2013/079231.

Des polysiloxanes comprenant de zones de rigidités distinctes sont également connus de Roger Piqueras Jover (« Variable rigidity surfaces for mechanobiology ») (polymérisation par voie chimique en utilisant la lithographie électronique pour augmenter le nombre de liaisons formées) ou de K. Tsougeni, A. Tserepi and E. Gogolides ( Microelectronic Engineering 84 (5-8), 1104-1108 (2007)) (PDMS photosensible polymérisé en UV profond).

Le procédé comprend une étape b) de dépôt de nanoobjets à la surface du gel.

Les nanoobjets sont choisis parmi :
- les protéines, les peptides et leurs mélanges,
- les polysaccharides, et
- les nanoparticules de métal.

Les nanoobjets peuvent être des bactéries.

Les nanoobjets ne sont pas des cellules. Dans un mode de réalisation, les nanoobjets ne sont pas des organismes vivants.

Le métal est notamment choisi parmi les métaux alcalins, les métaux alcalino-terreux, les lanthanides, les actinides, les métaux de transition et les métaux dits « pauvres », et est de préférence choisi parmi l'or, l'argent et l'indium.

Les protéines, peptides, polysaccharides et mélanges de ceux-ci sont les nanoobjets préférés, notamment des protéines et/ou peptides induisant une adhésion cellulaire via les intégrines, une telle protéine pouvant être de la fibronectine, du collagène, de la laminine ou des peptides du type RGD.

Le préfixe « nano » signifie que le diamètre moyen du nanoobjet est compris de 1 à 1000 nm, notamment de 2 à 500 nm, par exemple de 2 à 250 nm. Les nanoparticules d'or ont typiquement des diamètres moyens de 5 à 400 nm. Les nanoparticules d'argent ou d'indium ont typiquement des diamètres moyens de 2 à 10 nm

Le diamètre moyen du nanoobjet est donc supérieur au diamètre moyen des pores présents à la surface du gel (de l'ordre de 1 angström), ce qui permet que les nanoobjets restent à la surface du gel et ne s'enfoncent pas ou peu dans la matrice polymérique.

Le diamètre moyen des pores du gel peut être mesuré par diffusion de neutrons ou de rayons X aux petits angles.

Le diamètre moyen des protéines, peptides ou polysaccharides est typiquement mesuré par électrophorèse par gel. Le diamètre moyen des nanoparticules est typiquement mesurée par microscopie électronique à transmission ou à balayage (« transmission electron microscope » (TEM) et « scanning electron microscope » (SEM) en anglais).

Généralement, lors de l'étape b), les nanoobjets sont déposés sous forme d'un mélange comprenant les nanoobjets et un solvant. Le solvant de ce mélange peut être identique ou différent du solvant du gel. De préférence, le solvant du mélange est soluble dans le solvant du gel (soluble dans les conditions de l'étape b)). De manière préférée, le solvant du mélange est identique au solvant du gel.

Le mélange peut être colloïdal (les nanoobjets étant en suspension).

Le procédé peut comprendre, entre les étapes b) et c), une étape b1) consistant à laisser en contact les nanoobjets à la surface du gel, généralement pendant une durée de 1 min à 24 heures, notamment 1 min à 12 heures, par exemple 5 min à 1 heure. Lorsque les nanoobjets sont des protéines, cette étape correspond à une incubation. Généralement, quelle que soit la durée de cette mise en contact, il existe une différence de densité surfacique en nanoobjets entre les zones rigides et molles sur le gel obtenu à la fin du procédé. Une durée plus importante peut permettre de déposer plus de nanoobjets en surface (plus de nanoobjets à la fois sur les zones rigides et molles, mais la différence de densité surfacique en nanoobjets entre les zones rigides et molles existe quelle que soit cette durée).

Le procédé peut également comprendre, entre les étapes b) et c), (et après l'éventuelle étape b1) si elle est présente), une étape b2) consistant à éliminer une partie des nanoobjets de la surface du gel. Généralement, les nanoobjets ont été déposés sous forme d'un mélange comprenant les nanoobjets et un solvant, et l'étape b2) peut être mise en oeuvre en aspirant la solution surnageante au-dessus de la surface du gel, par exemple avec une pipette.

Le procédé comprend une étape c) d'évaporation du solvant du gel au moins jusqu'à ce que la variation du taux d'évaporation du solvant de la au moins une zone la moins rigide du gel ne soit pas constante dans le temps. Cette étape conduit à la migration des nanoobjets vers la au moins une zone la plus rigide du gel, et donc à l'obtention d'un gel dont la densité surfacique en nanoobjets d'au moins une zone la plus rigide parmi les au moins deux zones contiguës est supérieure à celle d'au moins une zone la moins rigide parmi les au moins deux zones contiguës.

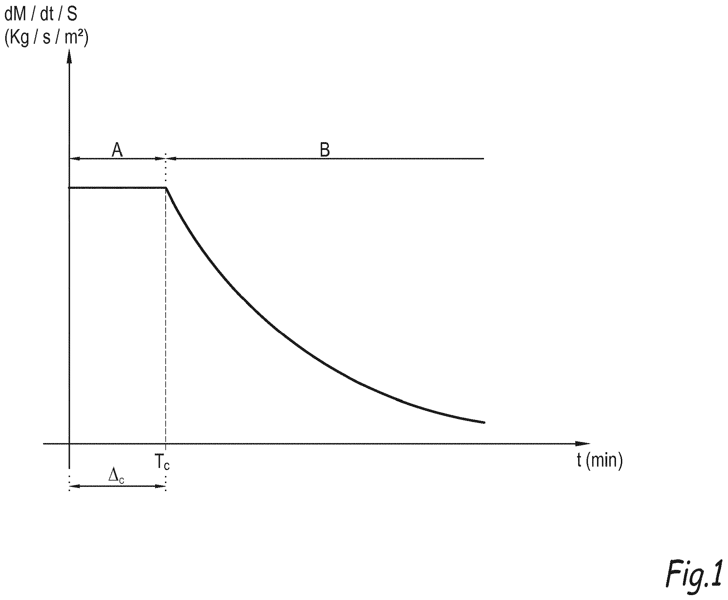

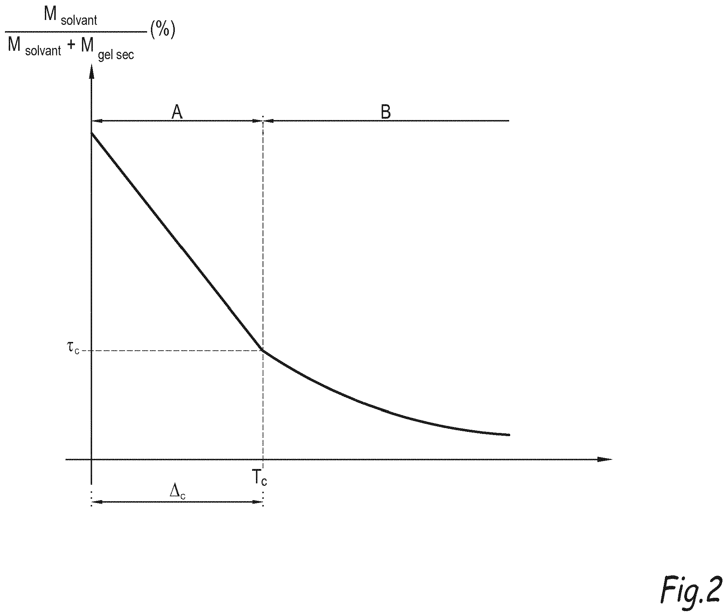

L'évaporation du solvant d'un gel de densité uniforme comprend deux régimes qui sont illustrés sur les figures 1 et 2.

Pendant la première période d'évaporation (« A » sur les figures 1 et 2), du solvant est éliminé en continu de la surface du gel par des forces capillaires et la teneur en solvant diminue à taux constant, ce qui s'explique par le fait que la surface du gel est suffisamment mouillée par le solvant et se comporte comme la surface d'un liquide. Son taux d'évaporation est égal à celui d'une surface liquide, qui dépend uniquement du gaz utilisé pour le séchage et du coefficient de transfert de la couche limite de la surface du gel.

Lorsque l'évaporation est poursuivie, le taux d'évaporation atteint un taux d'évaporation critique à un temps critique d'évaporation T_{c} qui marque la transition entre les première et deuxième périodes d'évaporation.

Pendant la deuxième période d'évaporation (« B » sur les figure 1 et 2), les forces de diffusion deviennent prédominantes par rapport aux forces de capillarité et l'élimination du solvant hors du gel est principalement contrôlée par la diffusion du solvant dans les pores du gel vers sa surface. Le taux d'évaporation du solvant n'est pas constant dans le temps. Il diminue jusqu'à atteindre un taux d'évaporation d'équilibre au-delà duquel le gel ne peut plus être séché.

Le temps critique d'évaporation T_{c} est le temps minimal à partir duquel le taux d'évaporation du solvant n'est pas constant dans le temps lorsque le solvant du gel est évaporé.

Le temps critique d'évaporation T_{c} est une caractéristique de chaque gel.

Il dépend de la nature du solvant et ses éventuels additifs (lorsque le solvant est une solution aqueuse, T_{c} dépend de la salinité, du pH, de la force ionique de cette solution). Par exemple, le temps critique d'évaporation T_{c} d'un gel dont le solvant comprend un agent viscosifiant est supérieur à celui d'un gel comprenant la même matrice polymérique et le même solvant, mais exempt d'agent viscosifiant. L'ajout d'un agent viscosifiant au solvant permet donc d'augmenter le temps critique d'évaporation T_{c} et la durée de la première période d'évaporation.

Lorsque le solvant est une solution aqueuse (le gel étant alors un hydrogel), l'évaporation du solvant est une déshydratation. Le temps critique d'évaporation T_{c} est alors le temps critique de déshydratation T_{c}, qui correspond au temps minimal à partir duquel le taux de déshydratation de la au moins une zone la moins rigide de l'hydrogel n'est pas constant dans le temps lorsque l'hydrogel est déshydraté.

Le temps critique d'évaporation T_{c} dépend également de la matrice polymérique, à savoir de la nature du polymère, mais aussi de sa porosité et donc de sa rigidité. Le temps critique d'évaporation T_{c} n'est donc pas identique entre les zones les plus rigides et les moins rigides du gel. Lors de l'étape c), l'évaporation est poursuivie au moins jusqu'à ce que la variation du taux d'évaporation du solvant de la au moins une zone la moins rigide du gel ne soit pas constante dans le temps. C'est donc la variation du taux d'évaporation du solvant de la au moins une zone la moins rigide du gel qui est considérée. De plus, le « au moins jusqu'à ce que la variation du taux d'évaporation du solvant ne soit pas constante dans le temps » signifie que l'évaporation du solvant de la au moins une zone la moins rigide du gel est poursuivie au moins jusqu'au temps critique d'évaporation T_{c}, pour que le taux d'évaporation de cette au moins une zone la moins rigide soit dans la deuxième période d'évaporation. Si on se réfère aux figures 1 et 2, lors de l'étape c), l'évaporation de la au moins une zone la moins rigide du gel est poursuivie au moins jusque T_{c}, ou plus loin, dans la deuxième période d'évaporation « B ». Aussi, l'étape c) revient généralement à évaporer le solvant du gel pendant une durée supérieure à la durée critique d'évaporation Δc, où la durée critique Δc est la durée entre le début d'évaporation et le temps critique d'évaporation T_{c} de la au moins une zone la moins rigide du gel.

Pour déterminer si la variation dans le temps du taux d'évaporation du solvant de la au moins une zone la moins rigide du gel est constant dans le temps ou non, il suffit de :
i. préparer un gel :
   - dont le polymère de la matrice polymérique est nature identique à celui de la matrice polymérique du gel mis en oeuvre dans le procédé selon l'invention,
   - dont le solvant est identique au solvant du gel mis en oeuvre dans le procédé selon l'invention (y compris l'éventuelle présence d'additifs comme d'un agent viscosifiant), et
   - dont la densité est égale à celle de la au moins une zone la moins rigide du gel utilisé dans le procédé selon l'invention.
   Le gel ainsi préparé a donc une densité uniforme, à la différence du gel utilisé dans le procédé selon l'invention. Le temps critique d'évaporation T_{c} du gel préparé est donc identique en toute zone du gel.
ii. puis évaporer le solvant du gel préparé, cette évaporation étant réalisée dans les mêmes conditions que celles de l'étape c), et en mesurant soit la teneur en solvant, soit le taux d'évaporation du solvant au cours du temps, et traçer :
   - la courbe du taux d'évaporation moyen en fonction du temps et déterminer le temps à partir duquel le taux d'évaporation n'est pas constant (figure 1), ou
   - la courbe de la teneur en solvant en fonction du temps et déterminer le temps à partir duquel la dérivée de la teneur en solvant n'est pas constante (figure 2, ou figure 1.3 p. 11 de la thèse de Thai Hong Vu soutenue en 2006 « Influence of Pore Size Distribution on Drying Behaviour of Porous Media by a Continuous Model », Fakultät für Verfahrens- und Systemtechnik der Otto-von-Guericke-Universität Magdeburg, Allemagne).

Ces courbes permettent donc de déterminer le temps critique d'évaporation T_{c} à partir duquel la deuxième période d'évaporation commence pour la au moins une zone la moins rigide du gel utilisé dans le procédé selon l'invention, et donc de déterminer à partir de quel moment, dans les conditions d'évaporation de l'étape c), la variation du taux d'évaporation du solvant de la au moins une zone la moins rigide du gel n'est pas constante dans le temps.

Le procédé selon l'invention, et la détermination du « au moins jusqu'à ce que la variation du taux d'évaporation du solvant de la au moins une zone la moins rigide du gel ne soit pas constante dans le temps », ne requièrent pas que les conditions de l'évaporation (nature du gaz, débit du gaz, pression, et/ou température du gaz) soient constantes lors de l'étape c). Il suffit uniquement que l'évaporation de l'étape ii) utilisée pour déterminer « au moins jusqu'à ce que la variation du taux d'évaporation du solvant de la au moins une zone la moins rigide du gel ne soit pas constante dans le temps » soit réalisée dans les mêmes conditions que celles de l'étape c).

Par « évaporation réalisée dans les mêmes conditions que celles de l'étape c) » on entend que, l'aire de la surface du gel utilisé à l'étape ii) est identique à ±10% à celle de la surface du gel mis en oeuvre dans l'étape c), l'épaisseur du gel utilisé à l'étape ii) est identique à ±10% à celle du gel mis en oeuvre dans le procédé selon l'invention, et que, à tout instant « t » de l'évaporation de l'étape ii) :
- le gaz servant à évaporer est identique à celui servant l'étape c) et/ou la pression est identique à ± 10% à celle de l'étape c) (si l'évaporation de l'étape c) est réalisée sous vide, la dépression est identique à ±10% pour l'évaporation de de ii)),
- la vitesse du gaz mis en contact avec le gel préparé est identique à ±10% au débit de gaz à l'instant t dans l'étape c), et
- la température du gaz servant à évaporer est identique à ± 2°C à la température du gaz servant à évaporer à l'instant t dans l'étape c).

Généralement, lors de l'étape c) :
- l'évaporation est réalisée par mise en contact du gel avec un gaz qui est de l'air ou un gaz inerte (tel que l'azote ou l'argon), de préférence de l'air, et/ou
- la pression est de 0,1 à 1 bar, de préférence à 1 bar, et/ou
- la température du gaz mis en contact avec le gel est de 4 à 90°C, notamment de 10 à 70°C, de préférence de 15 à 35°C, en particulier à température ambiante (20°C), et/ou
- la vitesse du gaz mis en contact est compris de 0 à 4 m/s, notamment de 0 à 1 m/s, de préférence de l'ordre de 0,45m/s (par exemple lorsque le gel est placé sous une hotte à flux laminaire),
ces conditions étant indépendamment constantes dans le temps ou variables dans le temps au cours de l'étape c).

De préférence, au moins jusqu'au temps critique d'évaporation T_{c} de la au moins une zone la moins rigide du gel, voire même pendant la durée de l'étape c), les conditions d'évaporation sont constantes dans le temps, c'est-à-dire que :
- lorsque l'évaporation est réalisée par mise en contact du gel avec un gaz :
   - la nature du gaz reste identique dans le temps,
   - le débit de gaz est constant à ±10%, et
   - la température du gaz est constante à ± 2°C, et
- la (dé)pression est constante à ±10% dans le temps (où (dé)pression signifie pression (P ≥ 1 bar)) ou dépression (P < 1 bar), pour une évaporation sous vide par exemple).

Généralement, au début de l'étape c) (lorsque l'évaporation est débutée), le gel a une teneur en solvant τₐ supérieure à la teneur en solvant τ_{C} du gel au temps critique d'évaporation T_{c} de la au moins une zone la moins rigide du gel. La teneur en solvant est ainsi telle que, au début de l'étape c), la variation du taux d'évaporation du solvant de la au moins une zone la moins rigide du gel est constante dans le temps. Au début de l'étape c), l'évaporation de la au moins une zone la moins rigide du gel est dans la première période d'évaporation définie ci-dessus. En pratique, cette condition est presque toujours vérifiée lorsque les nanoobjets sont déposés sous forme d'un mélange comprenant les nanoobjets et un solvant.

L'invention repose sur la découverte que le contrôle du taux d'évaporation du solvant au moment de la fixation des nanoobjets sur la surface du gel ayant un gradient de rigidité (ou de porosité) permet de contrôler la distribution des nanoobjets et donc leur densité surfacique. Plus précisément :
- Si l'évaporation du solvant du gel est stoppée alors que la variation du taux d'évaporation du solvant de la au moins une zone la moins rigide du gel est constante dans le temps, la distribution des nanoobjets est homogène à la surface du gel,
- Si l'évaporation du solvant du gel est effectuée au moins jusqu'à ce que la variation du taux d'évaporation du solvant de la au moins une zone la moins rigide du gel ne soit pas constante dans le temps, les nanoobjets sont attirés sélectivement vers les régions les plus rigides, c'est-à-dire les régions de plus faible porosité.

La distribution finale des nanoobjets est ainsi contrôlée par le taux de d'évaporation du gel au moment de la fixation des nanoobjets.

Sans vouloir être liés à une théorie particulière, les inventeurs supposent que le contrôle de la densité surfacique s'expliquerait par ce qui suit. De par sa structure, le gel utilisé dans le procédé est un matériau poreux, dont le taux de gonflement varie avec la teneur en solvant. Suivant la teneur en solvant, un gel mis en contact avec des nanoobjets absorbe le solvant pour atteindre son gonflement d'équilibre. Ce faisant, il concentre les nanoobjets contenus dans le solvant à sa surface. Par exemple, dans le cas d'un hydrogel à base de polyacrylamide, les coefficients de diffusion de l'eau et de protéines sont respectivement de l'ordre de 10⁻¹⁰ et 10⁻¹² m²/s. Les protéines pénètrent ainsi au moins 10 fois plus lentement que l'eau dans un hydrogel de polyacrylamide. Le gonflement de l'hydrogel en présence d'une solution de nanoobjets va donc résulter en la concentration des nanoobjets à sa surface. La distribution des nanoobjets est à ce stade liée aux forces de convection associées au pompage des régions de différentes porosités de l'hydrogel, au poids des nanoobjets, aux forces interactions avec la surface et aux forces entropiques qui tendent à uniformiser la distribution des nanoobjets. Dans le cas d'un gel comprenant au moins deux zones contiguës de rigidités distinctes, le temps critique d'évaporation T_{c} varie spatialement, avec la porosité/la rigidité : le solvant des régions les plus rigides, dont les pores sont de plus petite taille, s'évapore plus lentement. En conséquence, par un phénomène de démouillage, les nanoobjets situés dans les régions les plus molles, où les pores sont de plus grande taille, sont entrainés par les forces de tension de surface vers les régions encore solvatées. Ainsi, lorsque le solvant d'un gel comprenant au moins deux zones contiguës de rigidités distinctes est évaporé au-delà du temps critique d'évaporation T_{c} de ses régions les plus poreuses/les moins rigides, les nanoobjets distribués à sa surface sont déplacés vers les régions les moins poreuses/les plus rigides. C'est ce phénomène que la présente invention utilise pour concentrer les nanoobjets dans les régions les plus rigides à la surface de gels comprenant au moins deux zones contiguës de rigidités distinctes.

L'effet de concentration des nanoobjets sur les régions les plus rigides/les moins poreuses est d'autant plus marqué que le gradient de rigidité/porosité est fort. Aussi, des gradients de rigidité inférieur à 0,1 kPa/µm entre les zones contiguës conduisent à des gels dont la différence de densité surfacique en nanoobjets est trop faible pour la plupart des applications. C'est pourquoi le procédé met en oeuvre des gels dont le gradient de rigidité est supérieur.

Le procédé repose donc sur le déplacement des nanoobjets vers les régions les moins poreuses/les plus rigides lors de l'évaporation. Afin d'éviter d'entraver ce déplacement, il est préférable qu'il n'y ait pas d'interaction adhésive entre la surface du gel et les nanoobjets, notamment que les nanoobjets et la matrice polymérique du gel ne soient pas liés par liaison covalente, et/ou par liaison adhésive, ce qui peut être fait par un choix approprié du polymère et des nanoobjets (et notamment des éventuelles fonctions qu'ils portent) pour que de telles liaisons n'existent pas. La présence de liaisons adhésives peut être caractérisée par AFM et mesure du profil de force. La force de tension de surface est généralement supérieure à la force d'adhésion telle que mesurée par AFM. La force de tension de surface nécessite de connaitre le ratio des tensions superficielles présent dans la loi de Young Dupré, ce ratio pouvant être mesuré par exemple par une expérience d'angle de contact à l'équilibre (sans flux de gaz, notamment d'air). La mesure de l'angle de contact permet alors de mesurer la force de tension de surface.

Il y avait un préjugé technique à surmonter pour parvenir au procédé selon l'invention. En effet, l'homme du métier évite d'évaporer le solvant du gel, car il s'attend à le dégrader, notamment par fissuration/fracturation. Des préjugés techniques supplémentaires existaient lorsque les nanoobjets sont des protéines. En effet, l'homme du métier évite généralement d'évaporer le solvant du gel, car il s'attend à dégrader les protéines si elles se retrouvent à sec. En effet, la plupart des fournisseurs de protéines recommandent d'éviter de sécher une protéine qui a été mise en solution afin d'éviter de la dénaturer. Or, de manière surprenante, une telle dénaturation n'est généralement pas observée dans le procédé selon l'invention.

Lors de l'étape c), au début de l'évaporation, la teneur en sels minéraux dans le solvant du gel est de préférence est inférieure à 6 g/L, notamment inférieure à 5 g/L, typiquement inférieure à 4 g/L, par exemple inférieure à 3 g/L, de préférence inférieure à 2 g/L, une teneur inférieure à 1,5 g/L, voire inférieure à 1,0 g/L, voire même inférieure à 0,5 g/L, étant particulièrement préférée. De préférence, lors de l'étape c), le solvant est exempt de sels minéraux.

Des sels de chlorure (NaCl, KCI, CaCl₂ et/ou MgCl₂), des sels de phosphate (Na₂HPO₄ et/ou K₂HPO₄), des sels de carbonate (NaHCO₃) sont des exemples de sels minéraux. Ceux-ci sont utilisés de façon usuelle dans des solutions aqueuses physiologiques et/ou tampon utilisées à titre de solvant dans des hydrogels et pour les protéines.

Généralement, l'utilisateur connait la teneur en sels minéraux au début de l'évaporation, car il connait la teneur en sels minéraux dans le gel fourni à l'étape a) et la teneur en sels minéraux éventuellement ajoutés lors de l'étape b) (ces sels minéraux pouvant notamment provenir du solvant du mélange comprenant les nanoobjets et un solvant déposé lors de l'étape b)). Si la teneur en sels minéraux est inconnue, elle peut être déterminée par chromatographie ionique.

Les dégradations par fissuration/fracturation du gel lors de l'évaporation ne sont généralement pas observées lorsque la teneur en sels minéraux est telle que mentionnée ci-dessus. Sans vouloir être liés à une théorie particulière, les inventeurs observent que les sels minéraux, présents à des teneurs supérieures, cristallisent lors de l'évaporation du solvant, ce qui conduit à la fissuration du gel, notamment lors de son regonflement en vue de son utilisation sous forme solvatée, et de manière générale, conduit à la présence de nombreux dépôts et cristaux inamovibles sur la surface. De préférence, lors de l'étape c), le solvant est exempt de composé susceptible de cristalliser dans les conditions de l'étape c).

De plus, l'homme du métier est habitué à utiliser des protéines dans des milieux physiologiques, qui sont des solutions aqueuses, généralement tamponnées, et dont la teneur en sels minéraux excède celle mentionnée ci-dessus. Utiliser un solvant dont la teneur en sel est telle que définie ci-dessus est très inhabituel pour l'homme du métier.

Le procédé peut comprendre, avant l'étape a), les étapes consistant à :
a0) fournir un gel ayant une teneur en solvant initiale τᵢ supérieure à la teneur en solvant τₐ, puis
a0') évaporer le solvant du gel jusqu'à la teneur en solvant initiale τₐ, ce par quoi un gel tel que défini à l'étape a) est obtenu.
L'évaporation de l'étape a0') est donc réalisée avant le dépôt des nanoobjets. Cette évaporation préalable permet de diminuer l'épaisseur de la couche de solvant à la surface du gel et ainsi de favoriser la migration par convection des nanoobjets vers la surface du gel lors de l'étape b) qui suit.

Lorsque les nanoobjets sont des protéines et/ou des peptides et/ou des polysaccharides, le procédé peut comprendre, après l'étape c), une étape d) de greffage covalent des protéines et/ou peptides et/ou polysaccharides sur le gel, ce qui permet de les immobiliser définitivement et d'éviter que les peptides et/ou protéines et/ou polysaccharides ne se déplacent à nouveau à la surface du gel, lors du rinçage du gel par exemple. Les protéines et/ou les peptides et/ou les polysaccharides déposés lors de l'étape b) peuvent avoir été modifiés préalablement pour qu'ils soient porteurs d'une fonction susceptible de réagir avec la matrice polymérique du gel.

Le procédé peut comprendre, après l'étape c) ou l'étape d) si elle est présente, une étape e) de rinçage avec un solvant. Ce solvant peut être identique ou différent du solvant du gel. L'étape de rinçage peut être répétée.

Le procédé peut comprendre, après l'étape c), ou, si elles sont présentes, après l'étape d) ou e), une étape f) de récupération du gel dont la densité surfacique en nanoobjets d'au moins une zone la plus rigide parmi les au moins deux zones contiguës est supérieure à celle d'au moins une zone la moins rigide parmi les au moins deux zones contiguës. La méthode pour mesurer la densité surfacique en nanoobjets est variable selon la nature des nanoobjets. Par exemple, lorsque les nanoobjets sont des protéines, on peut utiliser un anticorps primaire susceptible de reconnaitre ladite protéine, puis un anticorps secondaire susceptible de reconnaitre ledit anticorps primaire, cet anticorps secondaire étant lié à un fluorophore, puis analyser la densité surfacique par microscopie confocale de fluorescence. Lorsque les nanoobjets sont des nanoparticules, la densité surfacique peut être analysée par microscopie électronique à balayage.

Le procédé selon l'invention est facile à mettre en oeuvre. Il ne nécessite pas d'équipement complexe. Il ne nécessite pas d'évaporation dirigée telle que Langmuir Blodgett.

Il peut être mis en oeuvre avec des motifs pour les zones rigides et/ou molles de n'importe quelle taille et forme. La géométrie des motifs est à façon, contrairement aux dépôts obtenus avec un dispenseur de microgouttes.

Le procédé selon l'invention permet de concentrer les nanoobjets sur la(es) zone(s) la(es) plus rigide(s), ce qui serait très difficile à obtenir en utilisant les techniques de tamponnage, de pochoir, de dispenseur de microgouttes ou de laser qui nécessiteraient une étape d'alignement entre la texture en rigidité et la modulation de la chimie de surface, étape technologiquement exigeante: elle consisterait en l'alignement avec une résolution de l'ordre du micron des reliefs respectivement du tampon, des jours du pochoir, de la position de la buse du dispenseur de microgouttes ou du faisceau laser avec les motifs de rigidité déjà présents dans le gel, ce qui est technologiquement atteignable mais difficile et coûteux en temps.

Le procédé selon l'invention permet de maintenir l'intégrité du gel, y compris de ses zones les moins rigides. Au contraire, les technologies de tamponnage et de pochoir sont inadaptées pour les rigidités de l'ordre du kPa car elles dégradent physiquement la surface. Quant aux méthodes d'activation de surface à travers un photomasque, les UV utilisés pour activer la surface modifient la rigidité/porosité de l'hydrogel lui-même texturé par photopolymérisation. En effet, le procédé d'activation de surface requis pour greffer les molécules interdit le rinçage de l'hydrogel nécessaire pour retirer son photo-initiateur, et donc stabiliser ses propriétés mécaniques. Enfin, la texturation topographique de l'hydrogel pour piéger des nanoobjets n'a, à la connaissance des inventeurs, jamais été réalisée en préservant à la fois la topographie souhaitée et la porosité/rigidité locale, soit à cause du gonflement des hydrogels (les trous se rebouchent car les parois se déforment), soit car le procédé de création de la topographie se fait par laser et durcit localement l'hydrogel par détérioration thermique (croûte de surface).

Le procédé selon l'invention est peu coûteux. L'organisation de nanoobjets par gradient de porosité est une technologie bas coût en comparaison de l'achat d'un dispenseur de microgouttes.

Selon un deuxième objet, l'invention concerne le gel susceptible d'être obtenu par le procédé défini ci-dessus, ledit gel comprenant une matrice polymérique comprenant au moins deux zones contiguës de rigidités distinctes, la surface du gel étant au moins partiellement revêtue de nanoobjets, où la densité surfacique en nanoobjets d'au moins une zone la plus rigide parmi les au moins deux zones contiguës est supérieure à celle d'au moins une zone la moins rigide parmi les au moins deux zones contiguës.

Le gradient de concentration en nanoobjets entre la au moins une zone la plus rigide et la au moins une zone la moins rigide contigüe peut être quantifiée par transformée de Fourier du profil d'intensité. Le motif de la au moins une zone la plus rigide étant généralement périodique, comme la densité surfacique en nanoobjets n'est pas homogène, un pic lié à la répétition du motif émerge du bruit de mesure. Lorsque le motif n'est pas périodique, l'histogramme de l'intensité permet de conclure sur la différence de distribution surfacique en nanoobjets entre zone la plus rigide et zone la moins rigide : si la distribution surfacique en nanoobjets est uniforme, l'histogramme doit être gaussien. Si elle est hétérogène, on aura deux gaussiennes. Dans ce cas, le critère pour conclure que elle est hétérogène est le critère de Shannon : les deux pics doivent être séparés de plus de la somme des 1/2 largeurs de chaque gaussienne.

Ce gel a des applications très variables selon la nature des nanoobjets déposés en surface. Selon un troisième objet, l'invention concerne l'utilisation de ce gel comme capteur photonique (typiquement lorsque les nanoobjets sont des semiconducteurs) ou physico-chimique, par exemple comme capteur de pH (typiquement lorsque les nanoobjets sont des particules d'or) ou de température (typiquement lorsque les nanoobjets sont des particules de CdTe), comme capteur pour la détection d'analyte, comme puce à protéines ou à peptides (typiquement lorsque les nanoobjets sont des protéines et/ou des peptides), comme puces à cellules ou comme puce de capture à biomolécules.

L'invention concerne également :
- une méthode de positionnement de cellules pour le criblage de principes actifs pharmaceutiques comprenant la mise en contact de principes actifs pharmaceutiques avec le gel selon l'invention dans lequel les nanoobjets sont des peptides ou des protéines,
- une méthode de capture de biomolécules comprenant la mise en contact d'un milieu comprenant des biomolécules à capturer avec le gel selon l'invention dans lequel les nanoobjets sont des peptides ou des protéines,
- une méthode d'analyse comprenant la mise en contact d'un milieu comprenant un analyte à détecter avec le gel selon l'invention.

Les figures et exemples ci-dessous illustrent l'invention.
Figure 1 : Courbe schématique de la perte de masse par unité de surface par unité de temps (dM/dt/S) en kg/s/m² d'un gel de densité uniforme en fonction du temps en minutes.
Figure 2 : Courbe schématique de la teneur en solvant (Msolvant / (Msolvant + M gel sec)) (%) d'un gel de densité uniforme en fonction du temps en minutes.
Figure 3 : Courbe de la perte de masse par unité de surface par unité de temps (dM/dt/S) en kg/s/m² d'un gel de densité uniforme de 3,3 kPa en fonction du temps en minutes.
Figure 4 : Motifs et profils de rigidité utilisés pour préparer les hydrogels comprenant deux zones contiguës de rigidités distinctes utilisés dans les exemples.
Figure 5 : Intensité de fluorescence (unité arbitraire) en fonction de la distance (µm) du gel obtenu par le procédé de l'exemple comparatif 1.
Figure 6 : Intensité de fluorescence (unité arbitraire) en fonction de la distance (µm) du gel obtenu par le procédé de l'exemple comparatif 2.
Figure 7 : A) Image d'immunofluorescence prise en microscopie confocale de la fibronectine fixée sur un hydrogel de polyacrylamide présentant une alternance de motifs triangulaires durs (10 kPa) de 20x100 µm espacés de 100 µm dans une matrice plus molle (3kPa) du gel obtenu par le procédé de l'exemple 3. Les variations de l'intensité de fluorescence dans l'épaisseur de l'hydrogel sont visualisées le long du pointillé blanc et reportées sur le panel sous-jacent.
   B) Intensité de fluorescence (unité arbitraire) en fonction de la distance (µm) du gel obtenu par le procédé de l'exemple 3.
Figure 8 : Intensité de fluorescence (unité arbitraire) en fonction de la distance (µm) du gel obtenu par le procédé de l'exemple 4.
Figure 9 : Intensité de fluorescence (unité arbitraire) en fonction de la distance (µm) du gel obtenu par le procédé de l'exemple comparatif 6.

### Exemples

Les exemples ont été réalisés avec des hydrogels de polyacrylamide. Les gels utilisés dans les exemples ci-dessous ont deux zones contiguës de rigidités distinctes. Pour chacun, le temps critique de déshydratation T_{c} de la zone la moins rigide est de quelques minutes et peut être déterminé comme suit.

Détermination de temps critique d'évaporation T_{c} de gels de densités uniformes.

Des gels ont été préparés à partir de compositions suivantes :

Composition:
- 10% acrylamide (250 µl de solution initialement à 40%)
- 0.5% N,N'-méthylènebisacrylamide (Bis) (250 µl de solution initialement à 2%)
- 0.2% Irgacure 819 w/v (Ciba, photo-initiateur)
- 1% propylamine (amorceur)
- eau déionisée (490 µl).

L'Irgacure 819 est pesé dans un flacon opaque aux UV. On y ajoute la propylamine. L'ensemble est chauffé à 50°C pendant 2 minutes. Après chauffage, on obtient une solution homogène, transparente. L'eau, l'acrylamide, et le bis acrylamide sont ajoutés rapidement. L'ensemble est homogénéisé doucement à la pipette, pour limiter l'incorporation d'oxygène.

Des gels de densité de 3,3, 11,8 ou 24,7 kPa ont ainsi été préparés.

L'évaporation a été réalisée à 21°C, sans flux de gaz (évaporation à l'air, dans la cage d'une balance de précision cagée (Denver Summit 110G/0,1MG) qui a permis une mesure en continu.

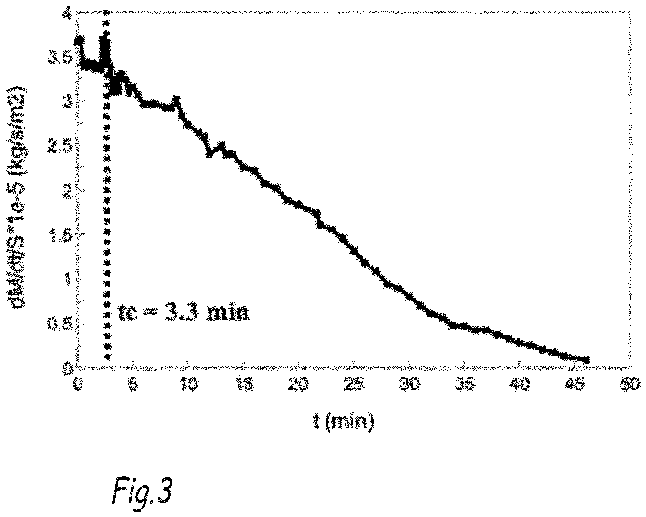

Dans les trois cas, on a tracé la courbe du taux d'évaporation moyen en fonction du temps pour une surface de 7 cm² et une épaisseur gonflée de l'ordre de 50 µm (figure 3 pour le gel de rigidité de 3,3 kPa), ce qui a permis de déterminer les temps critiques d'évaporation T_{c} fournis au tableau 1. En l'occurrence il s'agit de taux critique de déshydratation car les gels sont des hydrogels.

**Tableau 1 : temps critique de déshydratation T_{c} d'hydrogels acrylamide de densités uniformes**

| densité Y(kPa) | T_{c} (min) |
|---|---|
| 3,3 | 3,3±0,3 |
| 11,8 | 6,5±0,3 |
| 24,7 | 8,1±0,2 |

Les nanoobjets utilisés dans les exemples ci-dessous sont :
- soit des nanoparticules d'or, de diamètre 10 nm. Les nanoparticules sont simplement adsorbées à la surface du gel, sans greffage covalent ultérieur.
- Soit la fibronectine, couplée à un réticulant hétéro-bifonctionnel chargé d'assurer un greffage covalent entre la protéine et le gel par liaison des fonctions amines primaires des protéines et les fonctions amide du gel.

Exemple comparatif 1 : procédé dans lequel les protéines sont déposées sur un gel sec (τₐ < τ_{C}) et procédé exempt d'étape de déshydratation une fois que la protéine a été déposée : obtention d'une distribution uniforme de protéines.

Un hydrogel de polyacrylamide modulé en porosité et fixé de manière covalente à une lamelle de verre a été préparé par photolithographie à niveau de gris selon le procédé décrit dans la demande WO 2013/079231 et détaillé ci-après.

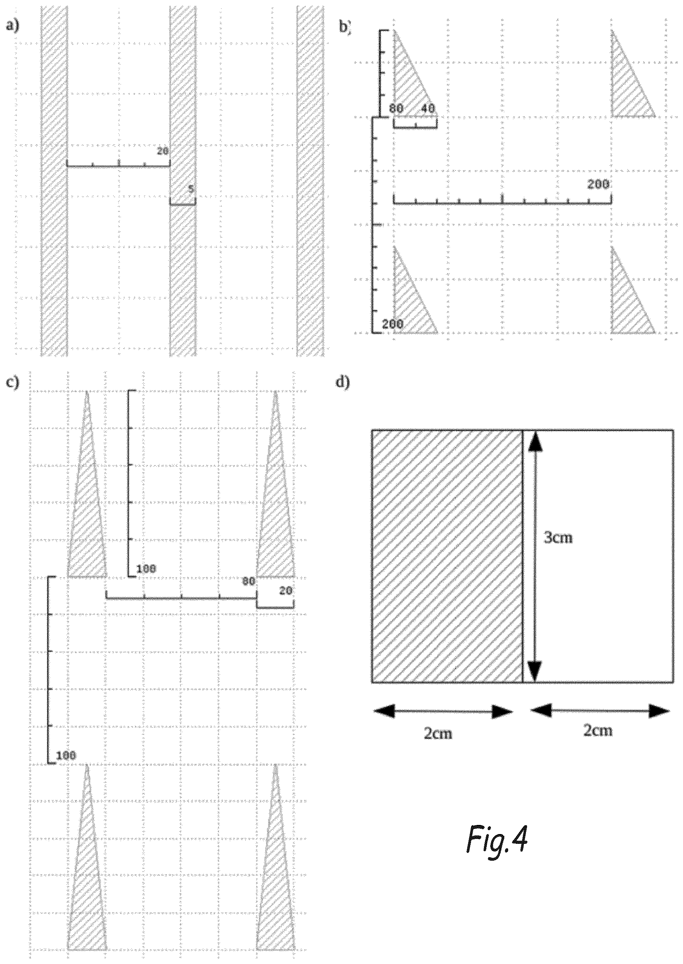

Le motif représenté sur la figure 4 par la référence « a » a été utilisé. L'hydrogel de polyacrylamide présentait une alternance de bandes dures (8.5 kPa) de 5 µm de large et de bandes plus molles (3.5 kPa) de 20µm de large.

### a) Préparation des lamelles de verre basales

La lamelle de verre basale, de diamètre 30 mm, est nettoyée dans une solution de 0,1 mol/L de soude pendant 10min. Elle est ensuite rincée intensivement à l'eau, puis à l'éthanol, et séchée à l'air. 500 µl d'une solution de silane comprenant 56 µl de Bind-Silane (GE Healthcare), 484µl d'acide acétique à 10%, et 14,46 mL d'éthanol ultra pur sont déposés sur la lamelle et frottés avec un chiffon tricoté de polyester jusqu'à disparition des traces de solution. On obtient ainsi une lamelle de verre présentant des fonctions aldéhydes à sa surface, qui permet le greffage covalent du gel de polyacrylamide.

### b) Préparation du masque à niveau de gris

Le masque à niveaux de gris est la copie maître du profil de rigidité qui sera transféré dans l'hydrogel. Ici est transféré le motif représenté sur la figure 4 par la référence « a ». Une lame de microscopie optique (26 mm x 76 mm) est lavée dans une solution d'eau oxygénée/acide sulfurique concentré en proportions 1:2, pendant 10 minutes. Sur cette lame, 1 nm de titane puis 9 nm de chrome sont déposés à l'aide d'un évaporateur à canon d'électrons de type Plassys. Une résine du type AZ1512HS (disponible chez Clariant) diluée à 50% dans son solvant AZ-EBR (qui est un solvant propylèneglycolmonométhyléther acétate) est déposée sur la lame côté chromé à l'aide d'une tournette à raison de 3000 tours/min pendant 30 secondes, moyennant quoi l'on obtient une épaisseur de résine de 600 nm. Elle est illuminée à travers un masque Sodalime présentant les motifs désirés. Après développement, la lame est placée dans un réacteur de gravure de type DPS, et gravée pendant 30 secondes à l'aide d'un traitement au chlore (2/3 Cl₂:1/3 O₂) sous une pression entre 1,33 et 3,33 Pa (10 et 25mTorr). La résine est retirée par un plasma O2 de 30 secondes dans le réacteur DPS. La lame est ensuite rendue hydrophobe par un traitement Optool (Daikin DSX) : immersion pendant 1 minute dans l'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

### c) Préparation de l'hydrogel

Composition:
- 10% acrylamide (250 µl de solution initialement à 40%)
- 0.5% N,N'-méthylènebisacrylamide (Bis) (250 µl de solution initialement à 2%)
- 0.2% Irgacure 819 w/v (Ciba, photo-initiateur)
- 1% propylamine (amorceur)
- eau déionisée (490 µl).

L'Irgacure 819 est pesé dans un flacon opaque aux UV. On y ajoute la propylamine. L'ensemble est chauffé à 50°C pendant 2 minutes. Après chauffage, on obtient une solution homogène, transparente. L'eau, l'acrylamide, et le bis acrylamide sont ajoutés rapidement. L'ensemble est homogénéisé doucement à la pipette, pour limiter l'incorporation d'oxygène. 30µL sont déposés sur la lamelle de verre de 30mm prétraitée selon le protocole ci-dessus. La lamelle est placée sur un porte-échantillon possédant des cales d'épaisseur qui maintiennent un espacement de 40µm entre la lamelle et le masque de chrome, déposé sur les cales d'épaisseur. L'ensemble (masque, solution, lamelle) est illuminé à l'aide d'une lampe fibrée Eleco UVP281 (2W/cm²) pendant 16s. Cet ensemble est ensuite plongé dans l'eau pour détacher le masque de l'hydrogel à l'aide d'une pince. L'hydrogel est rincé 3 fois avec de l'eau déionisée et conservé dans l'eau déionisée.

### d) Caractérisation de la rigidité de l'hydrogel

La variation relative de porosité des différentes régions de l'hydrogel est estimée par le biais de la mesure de la rigidité locale de l'hydrogel. La rigidité locale du gel est mesurée à l'aide d'un AFM en milieu aqueux (marque JPK). La résistance du gel à l'enfoncement de la pointe est enregistrée. Un scan de 34µm x 20µm est effectué. Les scans sont réalisés avec un pas de 1µm x 2µm. Il en résulte une série de courbes d'indentation. Chaque courbe est traitée selon le protocole du fabricant avec un modèle d'indentation élastique. Les rigidités obtenues sont dépendantes du temps d'illumination, de la forme et l'espacement des lignes. Elles sont de l'ordre de 8.5 kPa sur les régions rigides, et de 3.5 kPa sur les régions molles.

### e) Greffage covalent de fibronectine sur l'hydrogel

La protéine fibronectine est préalablement couplée au réticulant hetero-bifonctionnel sulfo-NHS-LC-Diazirine (Sulfosuccinimidyl 6-(4,4'-azipentanamido)hexanoate, ThermoScientific Pierce; nom commercial: sulfo-LC-SDA), avec un ratio molaire de 1/480: 5mg de fibronectine (Roche) sont dissous dans 2mL d'eau désionisée ultrapure, à 37°C pendant 30min. 1,2mg de sulfo-LC-SDA sont pesés à l'abri de la lumière et dissous dans la solution de fibronectine pendant 30min à température ambiante. Cette opération est répétée une seconde fois, conduisant au rapport molaire de 1/480. Ce protocole permet de faire réagir la fonction sulfo-NHS du sulfo-LC-SDA avec les groupements amine de la fibronectine en limitant l'hydrolyse du sulfo- LC-SDA. Le composé formé est une molécule de fibronectine couplée à une fonction photosensible diazirine. Le composé formé est dialysé à travers une membrane 6-8000 en chambre noire et à 4°C contre 2L de PBS+/+ 1x pendant 48h avec un changement du PBS au bout de 24h. Il est ensuite aliquoté en petits volumes (25 et 50µL) et conservé congelé à -20°C.

L'hydrogel préparé selon le protocole ci-dessus est déshydraté sous une hotte à flux laminaire vertical (Aura) à 26°C pendant une nuit (déshydratation de l'hydrogel en l'absence de fibronectine) jusqu'à obtention d'un gel sec. Le gel a donc une teneur en solvant τₐ inférieure à la teneur en solvant τ_{C} du gel à son temps critique d'évaporation T_{c}. Dans une pièce à éclairage sans UV, 800µL de solution de fibronectine conjuguée selon le protocole ci-dessus est préparée à une concentration de 2.2µg/mL dans de l'eau stérile déionisée, et est déposée à l'aide d'une pipette sur le gel. La solution de protéines est laissée incuber pendant 60min à 26°C sous la hotte à flux laminaire. La solution résiduelle est ensuite aspirée délicatement à l'aide d'une pipette, et le gel est immédiatement illuminé par la lampe UV ElecoUVP281 pendant 5min. Il est ensuite rincé délicatement 3 fois avec une solution de PBS+/+. Le gel fonctionnalisé est conservé hydraté dans une solution de PBS+/+, à 4°C.

### f) Caractérisation de la distribution des protéines greffées

La solution de PBS+/+ est aspirée du gel, et remplacée par une solution de saturation consistant en une solution de PBS+/+ 1x-Tween20 0.1%-BSA 2%, pendant 30min sous agitation lente à température ambiante (20°C). La solution de saturation est aspirée à l'aide d'une pipette et remplacée par une solution de 3µL d'anticorps polyclonal primaire anti fibronectine produit chez le lapin (Sigma-Aldrich, F3648) dilué dans 1.2mL de PBS+/+ 1x-Tween20 0.1%-BSA 2%. L'anticorps est incubé pendant 1h sous agitation lente à température ambiante. Il est ensuite révélé avec 1.2mL d'une solution contenant 0.6µL d'un anticorps secondaire couplé à l'Alexa Fluor 488 produit chez l'âne et dirigé contre le lapin (Molecular Probes, A21206), complété par une solution de PBS+/+ 1x-Tween20 0.1%-BSA 2% pendant 1h sous agitation lente à température ambiante et à l'abri de la lumière. La solution est ensuite retirée par aspiration et le gel est rincé 3 fois par 1,2mL de PBS+/+ 1x-Tween20 0.1%-BSA 2%. Le gel est ensuite conservé dans une solution de PBS+/+ 1x à 4°C et à l'abri de la lumière.

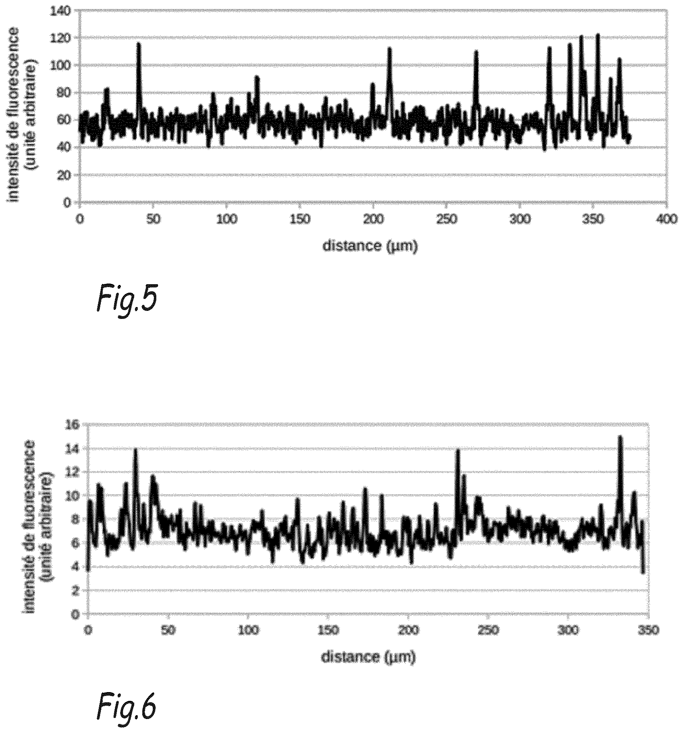

La caractérisation de la distribution des protéines greffées est effectuée par microscopie confocale de fluorescence (microscope Leica SP). Une pile d'images est acquise pour la longueur d'onde488nm avec un espacement entre les images de 0.28µm. La pile d'images est ensuite assemblée avec le logiciel ImageJ et des coupes sont extraites. Le profil d'intensité tracé représente la somme des intensités sur l'épaisseur de la pile, en chaque point de la surface du gel. Le profil de l'intensité de fluorescence de la coupe du bas montre une distribution de protéines indépendante du profil de rigidité/porosité (figure 5).

Exemple comparatif 2 : procédé dans lequel les protéines sont déposées sur un gel hydraté (τₐ > τ_{C}), mais procédé exempt d'étape de déshydratation une fois que la protéine a été déposée : obtention d'une distribution uniforme de protéines.

Dans cet exemple, l'hydrogel est utilisé partiellement hydraté, et la fixation de la protéine par illumination UV est effectué alors que la surface du gel est toujours humide (τₐ > τ_{C}). Ici est transféré le motif représenté sur la Figure 4 par la référence b.

### a) Préparation des lamelles de verre basales

Idem que Exemple 1 comparatif.a.

### b) Préparation du masque à niveau de gris

Ici est transféré le motif représenté sur la Figure 4 par la référence « b ».

Idem que Exemple 1. comparatif b, avec un dépôt de chrome de 14 nm.

### c) Préparation de l'hydrogel

Idem que Exemple 1 comparatif.c

Ici, l'ensemble (masque, solution, lamelle) est illuminé à l'aide d'une lampe fibrée Eleco UVP281 (2W/cm²) pendant 12s.

### d) Caractérisation de la rigidité de l'hydrogel

Idem que Exemple 1 comparatif.d. Un scan de 100µm x 60µm est effectué. Les scans sont réalisés avec un pas de 3.3µm. Les rigidités mesurées sont de l'ordre de 25 kPa sur les motifs, et de 3 kPa sur la matrice continue molle. Les motifs sont triangulaires rigides (25 kPa) de 80 x 40µm, de période 200µm, dans une matrice continue molle (3kPa).

### e) Greffage covalent de fibronectine sur l'hydrogel

Préparation de la protéine idem que Exemple 1 comparatif.e.

L'eau recouvrant l'hydrogel préparé selon le protocole est délicatement aspirée à l'aide d'une pipette. L'hydrogel est laissé déshydraté sous une hotte à flux laminaire (Aura) à 26°C pendant 10min, de manière que sa teneur en solvant τₐ soit supérieure à la teneur en solvant τ_{C} à son temps critique d'évaporation T_{c} (τₐ > τ_{C}).

Dans une pièce à éclairage sans UV, 800µL de solution de fibronectine conjuguée selon le protocole ci-dessus ajustée à une concentration de 2,2µg/mL dans de l'eau stérile déionisée est déposée à l'aide d'une pipette sur le gel. La solution de protéines est laissée incuber pendant une nuit à 26°C sous la hotte à flux laminaire fermée et éteinte, laissant ainsi le temps aux protéines d'approcher le gel sans que pour autant la surface ne se dessèche pendant cette durée (pas de déshydratation).

La solution résiduelle est ensuite aspirée délicatement à l'aide d'une pipette, et le gel est immédiatement illuminé par la lampe UV ElecoUVP281 pendant 5min. Il est ensuite rincé délicatement 3 fois avec une solution de PBS+/+. Le gel fonctionnalisé est conservé hydraté dans une solution de PBS+/+, à 4°C.

### f) Caractérisation de la distribution des protéines greffées

Idem que Exemple 1 comparatif.f.

Le profil de l'intensité de fluorescence montre une distribution uniforme de protéines (figure 6).

L'exemple 1 et l'exemple 2 montrent que la distribution finale des nanoobjets n'est pas dépendante du taux de gonflement qu'avait l'hydrogel lors du dépôt des nanoobjets (hydrogel sec à l'exemple comparatif 1 et hydraté à l'exemple comparatif 2). Ils montrent aussi que cette distribution n'est pas dépendante du temps de mise en contact entre les nanoobjets et la surface de l'hydrogel (incubation de 66 min à 26°C à l'exemple comparatif 1, une nuit à 26°C à l'exemple comparatif 2).

### Exemple 3 : Procédé selon l'invention - modulation de la densité surfacique de protéines sur des motifs plus rigides de tailles micrométriques

Ici est transféré le motif représenté sur la Figure 4 par la référence « c ».

### a) Préparation des lamelles de verre basales

Idem que Exemple 1 comparatif.a.

### b) Préparation du masque à niveau de gris

Idem que Exemple 2 comparatif.b.

### c) Préparation de l'hydrogel

Idem que Exemple 2 comparatif.c

### d) Caractérisation de la rigidité de l'hydrogel

Idem que Exemple 2 comparatif.d. Ici la rigidité du motif le moins poreux est d'environ 10 kPa, alors que la matrice continue entourant ces motifs est plus poreuse et a une rigidité de l'ordre de 3 kPa. La surface de l'hydrogel présente une alternance de motifs triangulaires durs (10 kPa) de 20 x100 µm espacés de 100 µm dans une matrice continue plus molle (3kPa).

### e) Greffage covalent de fibronectine sur l'hydrogel

Préparation de la protéine idem que Exemple 1 comparatif.e.

L'eau recouvrant l'hydrogel préparé selon le protocole ci-dessus est délicatement aspirée à l'aide d'une pipette (étape b2)).

L'hydrogel est laissé déshydraté sous une hotte à flux laminaire (Aura) à 26°C pendant 1h (étape a0')).

Dans une pièce à éclairage sans UV, 800µL de solution de fibronectine conjuguée selon le protocole ci-dessus ajustée à une concentration de 30,6µg/mL dans de l'eau stérile déionisée est déposée à l'aide d'une pipette sur le gel (étape b). La solution de protéines est laissée incuber pendant 60min sous la hotte à flux laminaire (étape b1)).

La solution résiduelle est ensuite aspirée délicatement à l'aide d'une pipette (étape b2)). Le gel mouillé de la solution de protéines est laissé déshydrater pendant 15 min sous la hotte à flux laminaire (Aura) à 26°C (étape c)).

Puis il est illuminé par la lampe UV ElecoUVP281 pendant 5min (étape d)). Il est ensuite rincé délicatement 3 fois avec une solution de PBS+/+ (étape e)). Le gel fonctionnalisé est conservé hydraté dans une solution de PBS+/+, à 4°C.

### f) Caractérisation de la distribution des protéines greffées

Idem que Exemple 1 comparatif.f.

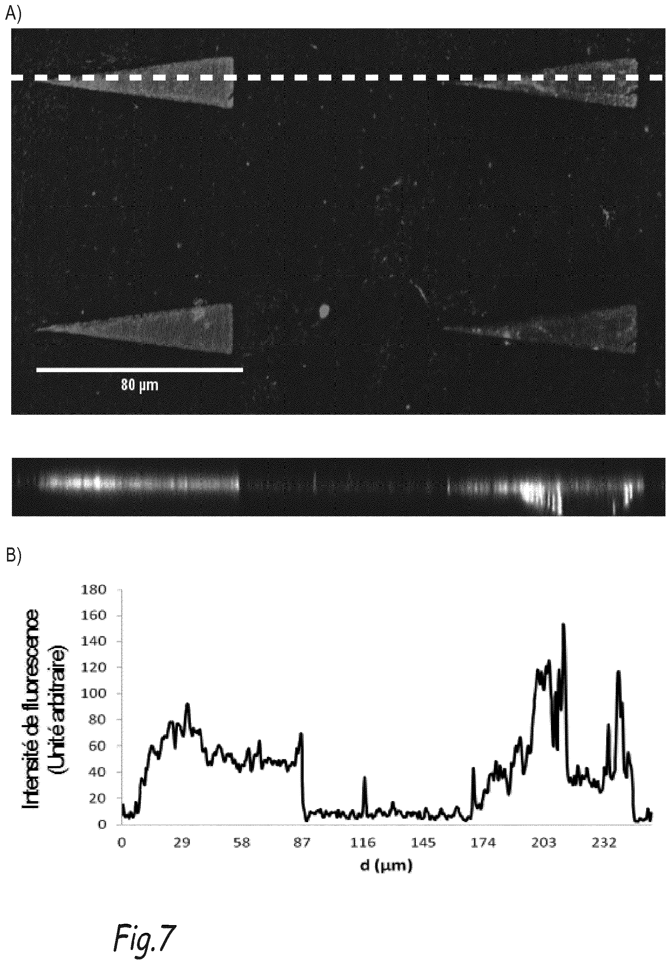

Le profil de l'intensité de fluorescence montre que les protéines sont beaucoup plus denses sur les motifs les plus durs/les moins poreux (figure 7).

### Exemple 4 : Procédé selon l'invention - modulation de la densité surfacique de protéines sur des motifs plus rigides de tailles millimétriques

Ici est transféré le motif représenté sur la Figure 4 par la référence « d ».

### a) Préparation des lamelles de verre basales

Idem que Exemple 1 comparatif.a.

### b) Préparation du masque à niveau de gris

Une lame de microscopie optique (26 mm x76 mm) est lavée dans une solution d'eau oxygénée/acide sulfurique concentré en proportion 1:1, pendant 20 minutes. Une plaque de silicium clivée est fixée sur la moitié gauche de la lame. Elle sert à cacher la partie transparente avant de procéder au dépôt. Ensuite 1nm de titane puis 19 nm de chrome sont déposés à l'aide d'un évaporateur à canon d'électrons de type Plassys. La lame est ensuite rendue hydrophobe par un traitement Optool (Daikin DSX) : immersion pendant 1 minute dans l'Optool dilué à 1/1000 dans du perfluorohexane ; puis la lame est laissée 1 heure dans de la vapeur d'eau à 80°C ; enfin elle est immergée sous agitation lente pendant 10 minutes dans du perfluorohexane.

### c) Préparation de l'hydrogel

Idem que Exemple 1. comparatif c. Ici, l'ensemble (masque, solution, lamelle) est illuminé à l'aide d'une lampe fibrée Eleco UVP281 (2W/cm2) pendant 36s.

### d) Caractérisation de la rigidité de l'hydrogel

Idem que Exemple 1. comparatif d. Le profil de rigidité est obtenu en scannant le gel perpendiculairement à la frontière de rigidité avec un pas de 5µm sur 400µm. Chaque point du profil est la moyenne de 5 mesures espacées de 20µm prises parallèlement à la frontière. L'hydrogel est composé d'une région dure de 3 cm² (40 kPa) et d'une région molle de même taille (1 kPa).

### e) Greffage covalent de fibronectine sur l'hydrogel

Préparation de la protéine idem que Exemple 1. comparatif e.

L'eau recouvrant l'hydrogel préparé selon le protocole ci-dessus est délicatement aspirée à l'aide d'une pipette.

L'hydrogel est déshydraté sous une hotte à flux laminaire (Aura) à 26°C pendant une nuit (étape a0')).

Dans une pièce à éclairage sans UV, 800µL de solution de fibronectine conjuguée selon le protocole ci-dessus ajustée à une concentration de 2,2µg/mL dans de l'eau stérile déionisée est déposée à l'aide d'une pipette sur le gel (étape b)).

La solution de protéines est laissée incuber pendant 60min sous la hotte à flux laminaire. (étape b1)) La solution résiduelle est ensuite aspirée délicatement à l'aide d'une pipette (étape b2)).

Le gel mouillé de la solution de protéines est laissé déshydrater pendant 60min sous la hotte à flux laminaire (Aura) à 26°C (étape c)).

Puis il est illuminé par la lampe UV ElecoUVP281 pendant 5min (étape d))..

Il est ensuite rincé délicatement 3 fois avec une solution de PBS+/+ (étape e)). Le gel fonctionnalisé est conservé hydraté dans une solution de PBS+/+, à 4°C.

### f) Caractérisation de la distribution des protéines greffées

Idem que Exemple 1 comparatif.f pour le marquage des protéines et l'obtention du profil d'intensité à l'échelle micrométrique. La caractérisation à l'échelle millimétrique est effectué en acquérant tous les millimètres une image de 375x375 µm en microscopie confocale. L'image 3D est transformée en une image 2D en moyennant l'intensité de chaque pixel sur l'épaisseur de la pile d'images. Puis une intensité moyenne est calculée à partir de l'image 2D.

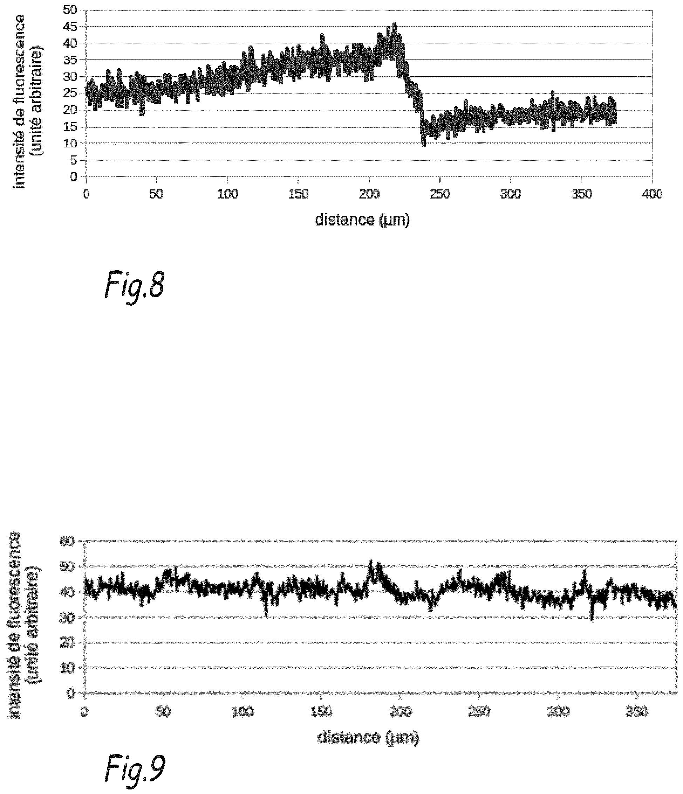

Le profil de l'intensité de fluorescence montre que la concentration surfacique des protéines est plus importante dans la région dure (figure 8).

### Exemple 5 : Procédé selon l'invention - modulation de la densité surfacique de nanoparticules d'or sur des motifs plus rigides de tailles micrométriques

### a) Préparation des lamelles de verre basales

Idem que Exemple 1 comparatif.a.

### b) Préparation du masque à niveau de gris

Idem que Exemple 2 comparatif.b. Les motifs choisis sont des triangles rectangles de taille 40 µm x 80 µm espacés de 200µm, issus des motifs présentés sur la Figure 4 « b ».

### c) Préparation de l'hydrogel

Idem que Exemple 2 comparatif.c

### d) Caractérisation de la rigidité de l'hydrogel

Idem que Exemple 2 comparatif.d.

### e) Adsorption des nanoparticules d'or

L'hydrogel préparé selon le protocole ci-dessus est déshydraté sous une hotte à flux laminaire vertical (Aura Mini) à 26°C pendant 60min (étape a0')).

Dans une pièce à éclairage sans UV, 800µL d'une solution de billes d'or de diamètre 10nm (BBI Solution, référence EM GC10) diluée à 5.2 1012 billes/ml dans de l'eau désionisée est déposée soit pendant 5 min, soit pendant 45 min sur la surface du gel (étape b)).

La solution résiduelle est ensuite aspirée délicatement à l'aide d'une pipette.

Le gel est laissé déshydrater sous la hotte à flux laminaire à 26°C pendant 4h (étape c)).

### f) Caractérisation de la distribution des nanoparticules d'or

La densité de surface des nanoparticules d'or est visualisée par microscopie électronique à balayage (MEB Zeiss Ultra Plus). Le gel avec les nanoparticules est recouvert d'un dépôt métallique de 3nm de platine (métalliseur BioRad SC500, 6,67 Pa (0.05Torr), 16mA, 30s). La visualisation est faite à 5keV, à une distance de travail de 7.5 mm pour l'échantillon ayant vu la suspension colloïdale 45 min, 7.6 mm pour celui correspondant à 5 min. Les deux images montrent une densité plus importante de nanoparticules sur le motif rigide qu'alentours. L'image pour l'échantillon 45 min montre quantitativement un nombre de nanoparticules plus important sur les régions dure et molle que l'échantillon 5 min (influence temps d'incubation).

### Exemple 6 comparatif : augmentation de Tc du glycérol comme agent viscosifiant

Le solvant de la solution de monomères qui permet la fabrication de l'hydrogel contient du glycérol.

L'augmentation de la viscosité du solvant ralentit l'évaporation et permet d'obtenir une chimie de surface uniforme malgré un temps d'attente avant fixation qui normalement induit une concentration des nanoobjets dans les régions les moins poreuses/les plus rigides.

Ici est transféré le motif représenté sur la Figure 4 par la référence « b ».

### a) Préparation des lamelles de verre basales

Idem que Exemple 1 comparatif.a.

### b) Préparation du masque à niveau de gris

Idem que Exemple 2 comparatif.b.

### c) Préparation de l'hydrogel

La composition de la solution à polymériser est la suivante :
- 10% acrylamide (250µl de solution initialement à 40%)
- 0.5% N,N'-méthylènebisacrylamide (Bis) (250µl de solution initialement à 2%)
- 0.2% Irgacure 819 w/v (Ciba, photo-initiateur)
- 1% propylamine (amorceur)
- eau déionisée (489 µl)
- 0.1 % glycérol (1 µL)

Puis idem que Exemple 2 comparatif.c.

### d) Caractérisation de la rigidité de l'hydrogel

Idem que Exemple 2 comparatif.d.

L'hydrogel présente des motifs triangulaires rigides (25 kPa) de 80 x 40µm, de période 200µm, dans une matrice continue molle (3kPa).

### e) Greffage covalent de fibronectine sur l'hydrogel

Préparation de la protéine idem que Exemple 1 comparatif.e.

L'eau recouvrant l'hydrogel préparé selon le protocole ci dessus est délicatement aspirée à l'aide d'une pipette.

L'hydrogel est laissé déshydraté sous une hotte à flux laminaire (Aura) à 26°C pendant 5min.

Dans une pièce à éclairage sans UV, 800µL de solution de fibronectine conjuguée selon le protocole ci-dessus ajustée à une concentration de 2.2µg/mL dans de l'eau stérile déionisée est déposée à l'aide d'une pipette sur le gel.

La solution de protéines est laissée incuber pendant 60min.

La solution résiduelle est ensuite aspirée délicatement à l'aide d'une pipette.

Le gel est déshydraté pendant 5min sous une hotte à flux laminaire (Aura), puis illuminé par la lampe UV ElecoUVP281 pendant 5min. Il est ensuite rincé délicatement 3 fois avec une solution de PBS+/+.

Le gel fonctionnalisé est conservé hydraté dans une solution de PBS+/+, à 4°C.

### f) Caractérisation de la distribution des protéines greffées

Idem que Exemple 1 comparatif.f.

Le profil de l'intensité de fluorescence de la coupe du bas montre une distribution uniforme de protéines (figure 9).

Cet exemple 6 montre que le temps critique de déshydration T_{c} peut être augmenté par l'ajout d'un adjuvant qui augmente la viscosité du solvant et limite ainsi l'évaporation. Dans cet exemple, on obtient un greffage uniforme de protéines malgré l'existence d'une étape de déshydratation après le dépôt de protéines. La durée de déshydratation n'était pas suffisante. Le taux critique de déshydratation n'a pas été atteint.

## Revendications

1. Procédé de dépôt de nanoobjets sur la surface d'un gel comprenant une matrice polymérique comprenant au moins deux zones contiguës de rigidités distinctes, ledit procédé comprenant les étapes de :
a) fournir un gel comprenant une matrice polymérique et un solvant au sein de la matrice polymérique, la matrice polymérique formant un réseau tridimensionnel susceptible de gonfler en présence dudit solvant, où la solubilité de la matrice polymérique à 1 bar et 25°C dans le solvant est inférieure à 1 g/L, la matrice polymérique comprenant au moins deux zones contiguës de rigidités distinctes présentant un gradient de rigidité supérieur ou égal à 0,1 kPa/µm, puis
b) déposer des nanoobjets dont le diamètre moyen est de 1 à 1000 nm, mesuré selon la méthode décrite dans la description, à la surface du gel, puis
c) évaporer le solvant du gel au moins jusqu'à ce que la variation du taux d'évaporation du solvant de la au moins une zone la moins rigide du gel ne soit pas constante dans le temps, ce par quoi les nanoobjets migrent vers la au moins une zone la plus rigide du gel et un gel est obtenu dont la surface est au moins partiellement revêtue de nanoobjets, et où la densité surfacique en nanoobjets d'au moins une zone la plus rigide parmi les au moins deux zones contiguës est supérieure à celle d'au moins une zone la moins rigide parmi les au moins deux zones contiguës, la détermination dudit au moins jusqu'à ce que la variation du taux d'évaporation du solvant de la au moins une zone la moins rigide du gel ne soit pas constante dans le temps étant réalisée selon la méthode décrite de la page 10, ligne 10 à la page 11, ligne 4 de la description ; et lesdits nanoobjets étant choisis parmi les polysaccharides, les protéines, les peptides, et mélanges de ceux-ci, et les nanoparticules de métal et lesdits nanoobjets n'étant pas des cellules.

2. Procédé selon la revendication 1, dans lequel les nanoobjets ne sont pas des organismes vivants.

3. Procédé selon la revendication 1 ou 2, dans lequel la matrice polymérique du gel comprend un polymère choisi parmi :
- les polyacrylamides ;
- les polyéthylène glycols, polypropylène glycols et copolymères d'éthylène glycol ou de propylène glycol, ceux-ci comprenant éventuellement des motifs issus de la polymérisation de composés (meth)acrylates;
- les polysaccharides, comprenant éventuellement des motifs répétitifs issus de la polymérisation de composés (meth)acrylates ;
- les (co)polymères issus de la polymérisation de composés diacrylates et/ou (méth)acrylates ;
- les alcools polyvinyliques comprenant des motifs répétitifs issus de la polymérisation de composés (meth)acrylates;
- les dextranes comprenant des motifs répétitifs issus de la polymérisation de composés (méth)acrylates ;
- les polyfumarates de propylène et les poly (fumarate de propylène-co-éthylène glycol) ;
- les polysiloxanes, comme le poly(dimethylsiloxane) ; et
- les combinaisons de ceux-ci,
de préférence les polyacrylamides, par exemple issus de la polymérisation de l'acrylamide et du N,N'-méthylènebisacrylamide.

4. Procédé selon l'une quelconque des revendications 1 à 3, dans lequel le solvant présent au sein de la matrice polymérique du gel est une solution aqueuse.

5. Procédé selon l'une quelconque des revendications 1 à 3, dans lequel le solvant présent au sein de la matrice polymérique du gel est choisi parmi le pentane, la triéthylamine, la diisopropylamine et le xylène et la matrice polymérique comprend du poly(dimethylsiloxane).

6. Procédé selon l'une quelconque des revendications 1 à 5, dans lequel le solvant comprend un agent viscosifiant.

7. Procédé selon l'une quelconque des revendications 1 à 6, dans lequel les nanoobjets sont choisis parmi les polysaccharides, les protéines, les peptides et les mélanges de ceux-ci, notamment les protéines et/ou peptides induisant une adhésion cellulaire via les intégrines, de préférence la fibronectine, le collagène, la laminine ou les peptides du type RGD.

8. Procédé selon la revendication 7, comprenant, après l'étape c), une étape d) de greffage covalent des protéines et/ou peptides et/ou polysaccharides sur le gel.

9. Gel susceptible d'être obtenu par le procédé selon l'une quelconque des revendications 1 à 8, ledit gel comprenant une matrice polymérique comprenant au moins deux zones contiguës de rigidités distinctes, la surface du gel étant au moins partiellement revêtue de nanoobjets, les nanoobjets étant choisis parmi les polysaccharides, les protéines, les peptides, et mélanges de ceux-ci, et les nanoparticules de métal et lesdits nanoobjets n'étant pas des cellules, où la densité surfacique en nanoobjet d'au moins une zone la plus rigide parmi les au moins deux zones contiguës est supérieure à celle d'au moins une zone la moins rigide parmi les au moins deux zones contiguës.

10. Utilisation du gel selon la revendication 9 comme capteur photonique ou physico-chimique, comme capteur pour la détection d'analyte, comme puce à protéines ou à peptides, comme puces à cellules ou comme puce de capture à biomolécules.

## Patentansprüche

1. Verfahren zum Abscheiden von Nanoobjekten auf der Oberfläche eines Gels, das eine Polymermatrix umfasst, die mindestens zwei aneinandergrenzende Bereiche mit unterschiedlichen Starrheiten umfasst, wobei das Verfahren die folgenden Schritte umfasst:
a) Bereitstellen eines Gels, das eine Polymermatrix und ein Lösungsmittel innerhalb der Polymermatrix umfasst, wobei die Polymermatrix ein dreidimensionales Netzwerk bildet, das in Gegenwart des Lösungsmittels aufquellen kann, wobei die Löslichkeit der Polymermatrix bei 1 bar und 25 °C in dem Lösungsmittel weniger als 1 g/l beträgt,
wobei die Polymermatrix mindestens zwei aneinandergrenzende Bereiche mit unterschiedlichen Starrheiten umfasst, die einen Starrheitsgradienten von größer als oder gleich 0,1 kPa/µm aufweisen, und dann
b) Abscheiden von Nanoobjekten mit einem mittleren Durchmesser von 1 bis 1000 nm, gemessen gemäß dem in der Beschreibung beschriebenen Verfahren, an der Oberfläche des Gels, und dann
c) Verdampfen des Lösungsmittels aus dem Gel mindestens so lange, bis die Änderung der Verdampfungsrate des Lösungsmittels aus dem mindestens einen am wenigsten starren Bereich des Gels zeitlich nicht konstant ist, wodurch die Nanoobjekte zu dem mindestens einen starrsten Bereich des Gels wandern und ein Gel erhalten wird, dessen Oberfläche zumindest teilweise mit Nanoobjekten beschichtet ist, und wobei die Flächendichte an Nanoobjekten des mindestens einen starrsten Bereichs der mindestens zwei aneinandergrenzenden Bereiche größer ist als die des mindestens einen am wenigsten starren Bereichs der mindestens zwei aneinandergrenzenden Bereiche, wobei die Bestimmung des mindestens bis die Änderung der Verdampfungsrate des Lösungsmittels des mindestens einen am wenigsten starren Bereichs des Gels, die zeitlich nicht konstant ist, gemäß dem von Seite 10, Zeile 10 bis Seite 11, Zeile 4 der Beschreibung beschriebenen Verfahren durchgeführt wird; und
wobei die Nanoobjekte ausgewählt sind aus Polysacchariden, Proteinen, Peptiden und Mischungen davon, und wobei die Metallnanopartikel und die Nanoobjekte keine Zellen sind.

2. Verfahren nach Anspruch 1, wobei es sich bei den Nanoobjekten nicht um lebende Organismen handelt.

3. Verfahren nach Anspruch 1 oder 2, wobei die Polymermatrix des Gels ein Polymer umfasst, das ausgewählt ist aus:
- Polyacrylamiden;
- Polyethylenglykolen, Polypropylenglykolen und Copolymeren von Ethylenglykol oder Propylenglykol, wobei diese gegebenenfalls Einheiten enthalten, die aus der Polymerisation von (Meth)acryllatverbindungen stammen;
- Polysacchariden, die gegebenenfalls wiederkehrende Einheiten aus der Polymerisation von (Meth)acrylatverbindungen enthalten;
- (Co)polymeren, die aus der Polymerisation von Diacrylat- und/oder (Meth)acrylatverbindungen stammen;
- Polyvinylalkoholen, die wiederkehrende Einheiten umfassen, die aus der Polymerisation von (Meth)acryllatverbindungen stammen;
- Dextranen, die wiederkehrende Einheiten enthalten, die aus der Polymerisation von (Meth)acrylatverbindungen stammen;
- Polypropylenfumaraten und Poly(propylenfumarat-co-ethylenglycol);
- Polysiloxanen wie Poly(dimethylsiloxan); und
- Kombinationen davon,
vorzugsweise Polyacrylamide, die z. B. aus der Polymerisation von Acrylamid und N,N'-Methylenbisacrylamid stammen.

4. Verfahren nach einem der Ansprüche 1 bis 3, wobei das in der Polymermatrix des Gels vorhandene Lösungsmittel eine wässrige Lösung ist.

5. Verfahren nach einem der Ansprüche 1 bis 3, wobei das in der Polymermatrix des Gels vorhandene Lösungsmittel aus Pentan, Triethylamin, Diisopropylamin und Xylol ausgewählt ist und die Polymermatrix Poly(dimethylsiloxan) umfasst.

6. Verfahren nach einem der Ansprüche 1 bis 5, wobei das Lösungsmittel ein viskosifizierendes Mittel umfasst.

7. Verfahren nach einem der Ansprüche 1 bis 6, wobei die Nanoobjekte ausgewählt sind aus Polysacchariden, Proteinen, Peptiden und Mischungen davon, insbesondere Proteinen und/oder Peptiden, die die Zelladhäsion über Integrine induzieren, vorzugsweise Fibronektin, Kollagen, Laminin oder Peptide vom RGD-Typ.

8. Verfahren nach Anspruch 7, das nach Schritt c) einen Schritt d) zur kovalenten Pfropfung der Proteine und/oder Peptide und/oder Polysaccharide auf das Gel umfasst.

9. Gel, das durch das Verfahren nach einem der Ansprüche 1 bis 8 erhalten werden kann, wobei das Gel eine Polymermatrix umfasst, die mindestens zwei aneinandergrenzende Bereiche mit unterschiedlichen Starrheiten umfasst, wobei die Oberfläche des Gels mindestens teilweise mit Nanoobjekten beschichtet ist, wobei die Nanoobjekte ausgewählt sind aus Polysacchariden, Proteinen, Peptiden und Mischungen davon, und wobei die Metallnanopartikel und die Nanoobjekte keine Zellen sind, wobei die Flächendichte an Nanoobjekten von mindestens einem starrsten Bereich der mindestens zwei aneinandergrenzenden Bereiche größer ist als die von mindestens einem am wenigsten starren Bereich der mindestens zwei aneinandergrenzenden Bereiche.

10. Verwendung des Gels nach Anspruch 9 als photonischer oder physikalisch-chemischer Sensor, als Sensor für den Nachweis von Analyten, als Protein- oder Peptidchip, als Zellchip oder als Biomolekül-Erfassungschip.

## Claims

1. Method for depositing nanoobjects on the surface of a gel comprising a polymeric matrix comprising at least two contiguous zones with distinct rigidities, said method comprising the steps of:
a) providing a gel comprising a polymeric matrix and a solvent in the polymeric matrix, the polymeric matrix forming a three-dimensional lattice able to swell in the presence of said solvent, where the solubility of the polymeric matrix at 1 bar and 25°C in the solvent is less than 1 g/L,
the polymeric matrix comprising at least two contiguous zones with distinct rigidities having a rigidity gradient greater than or equal to 0.1 kPa/µm, then
b) depositing nanoobjects the mean diameter of which is from 1 to 1000 nm, measured in accordance with the method described in the description, on the surface of the gel, then
c) evaporating the solvent from the gel at least until the variation in the degree of evaporation of the solvent in the at least one least rigid zone of the gel is not constant over time, by means of which the nanoobjects migrate towards the at least one most rigid zone of the gel and a gel is obtained the surface of which is at least partially coated with nanoobjects, and where the surface density of nanoobjects of at least one most rigid zone among the at least two contiguous zones is greater than that of at least one least rigid zone among the at least two contiguous zones, the determination of said at least until the variation in the degree of evaporation of the solvent of the at least one least rigid zone of the gel is not constant over time being implemented in accordance with the method described on page 10 line 10 to page 11 line 4 of the description; and
said nanoobjects being selected from polysaccharides, proteins, peptides and mixtures thereof, and the metal nanoparticles and said nanoobjects not being cells.

2. Method according to claim 1, wherein the nanoobjects are not living organisms.

3. Method according to claim 1 or 2, wherein the polymeric matrix of the gel comprises a polymer selected from:
- polyacrylamides;
- polyethylene glycols, polypropylene glycols and copolymers of ethylene glycol or polypropylene glycol, these optionally comprising units resulting from the polymerisation of methacrylate compounds;
- polysaccharides, optionally comprising repeat units resulting from the polymerisation of methacrylate compounds;
- (co)polymers resulting from the polymerisation of diacrylate and/or methacrylate compounds;
- polyvinyl alcohols comprising repeat units resulting from the polymerisation of methacrylate compounds;
- dextrans comprising repeat units resulting from the polymerisation of methacrylate compounds;
- propylene polyfumarates and polypropylene fumarate-co-ethylene glycol);
- polysiloxanes, such as poly(dimethylsiloxane); and
- combinations thereof,
preferably polyacrylamides, for example resulting from the polymerisation of acrylamide and N, N'-methylenebisacrylamide.

4. Method according to any one of claims 1 to 3, wherein the solvent present in the polymeric matrix of the gel is an aqueous solution.

5. Method according to any one of claims 1 to 3, wherein the solvent present in the polymeric matrix of the gel is selected from pentane, triethylamine, diisopropylamine and xylene and the polymeric matrix comprises poly(dimethylsiloxane).

6. Method according to any one of claims 1 to 5, wherein the solvent comprises a viscosifying agent.

7. Method according to any one of claims 1 to 6, wherein the nanoobjects are selected from polysaccharides, proteins, peptides and mixtures thereof, in particular proteins and/or peptides causing cell adhesion via the integrins, preferably fibronectin, collagen, laminin or peptides of the RGD type.

8. Method according to claim 7, comprising, after step c), a step d) of covalent grafting of the proteins and/or peptides and/or polysaccharides on the gel.

9. Gel able to be obtained by the method according to any one of claims 1 to 8, said gel comprising a polymeric matrix comprising at least two contiguous zones with distinct rigidities, the surface of the gel being at least partially coated with nanoobjects, the nanoobjects being selected from polysaccharides, proteins, peptides and mixtures thereof, and the metal nanoparticles and said nanoobjects not being cells, where the surface density of nanoobjects of at least one most rigid zone among the at least two contiguous zones being greater than that of at least one least rigid zone among the at least two contiguous zones.

10. Use of the gel according to claim 9 as photon or physicochemical sensor, as sensor for analyte detection, as protein or peptide chip, as cell chips or as biomolecule capture chip.
